(19) Europäisches Patentamt
European Patent Office
Office européen des brevets

(11) **EP 3 806 725 B1**

(12) **EUROPEAN PATENT SPECIFICATION**

(45) Date of publication and mention
of the grant of the patent:
**27.09.2023 Bulletin 2023/39**

(21) Application number: **19745208.9**

(22) Date of filing: **16.07.2019**

(51) International Patent Classification (IPC):
**A61B 5/283** (2021.01)    **A61B 5/0215** (2006.01)
**A61B 5/02** (2006.01)    **A61B 5/06** (2006.01)
**A61B 5/00** (2006.01)    **A61B 5/026** (2006.01)

(52) Cooperative Patent Classification (CPC):
**A61B 5/0215; A61B 5/02007; A61B 5/02158;
A61B 5/026; A61B 5/065; A61B 5/283;
A61B 5/6851; A61B 5/6852; A61B 5/7264;
A61B 5/7267; A61B 5/7289;** A61B 5/287;
A61B 2562/0247; A61B 2562/043; G16H 50/20;

(Cont.)

(86) International application number:
**PCT/GB2019/051978**

(87) International publication number:
**WO 2020/016565 (23.01.2020 Gazette 2020/04)**

(54) **AN INTRACORONARY WIRE AND SYSTEM FOR EVALUATING INTRACORONARY FLOW**

INTRAKORONARDRAHT UND SYSTEM ZUR BEWERTUNG DES INTRAKORONAREN FLUSSES

FIL INTRACORONAIRE ET SYSTÈME D'ÉVALUATION D'UN FLUX INTRACORONAIRE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **16.07.2018 GB 201811610**

(43) Date of publication of application:
**21.04.2021 Bulletin 2021/16**

(73) Proprietor: **Cerebria Limited
London EC1V 9EE (GB)**

(72) Inventor: **DAVIES, Justin
London Greater London EC1V 9EE (GB)**

(74) Representative: **Forresters IP LLP
Skygarden
Erika-Mann-Straße 11
80636 München (DE)**

(56) References cited:
**US-A- 5 509 411      US-A1- 2004 116 816
US-A1- 2015 112 901    US-A1- 2018 116 723**

(52) Cooperative Patent Classification (CPC): (Cont.)
   G16H 50/70

**Description**

Field of the invention

This invention relates to an intracoronary wire according to independent claim 1. Preferred embodiments are defined by dependent claims 2-15.

One aspect of the disclosure relates to a particular form of intracoronary wire with the capability to obtain pressure-based data and electrocardiogram (ECG) data.

Another aspect of the disclosure relates to a system for utilising data derived from an intracoronary wire.

Background of the invention

False positive stress test results and/or failure of clinical judgment can indicate the presence of stenoses. However, 30-40% of patients with signs/symptoms of myocardial ischemia (MI - angina pectoris) have normal epicardial coronary arteries with chamber and vessel limiting stenoses. Stenoses can be imaged by conducting a coronary angiogram (where a contrast material is used to highlight flow and identify the exact location and severity of any coronary arterial disease (CAD)). In a substantial proportion of patients with signs/symptoms of myocardial ischemia, the signs/symptoms develop due to the presence of coronary microvascular dysfunction (CMD).

CMD is dysfunction of the microcirculation and is defined as impaired vasodilation of coronary arterioles in response to stress leading to in adequate increase in myocardial blood flow from rest to stress. Functional and/or structural microvascular abnormalities contribute to this pathology. Prevalence of CMD among patients with chest pain and normal epicardial coronary arteries (or with chamber and vessel limiting stenoses) is reported to be as high as 64%. In another words, more than 60% of these patients with normal or near normal coronary angiogram and chest pain show microvascular abnormalities.

In addition, abnormalities in coronary microvascular vasomotion are shown to be directly associated with myocardial ischemia. Anginal scores (based on a patient's symptoms) reflecting extent of myocardial ischemia are shown to be directly related to myocardial perfusion reserve at cardiac magnetic resonance imaging in patients with normal or near normal epicardial coronary arteries and microvascular disease (MVD). When CMD is due to MVD, then this is disease of the micro vasculature.

Importantly, the presence of microvascular disease (abnormal / decreased coronary blood flow reserve) was found to be an independent predictor of adverse cardiovascular events, including hospitalization for worsening angina, MI, congestive heart failure, stroke, revascularization, other vascular events, and death.

In brief, there is a large number of patients with signs and symptoms of ischemia despite no evidence of obstructive atherosclerosis on coronary angiogram indicating presence of microvascular dysfunction/disease. Prognosis of this patient group is significantly worse compared with a background population without known coronary artery disease. However, there is no highly specific, straightforward (standardized, easy to use and interpret) and widely established technique for identification of these patients.

Diagnosis of coronary microvascular dysfunction leading to coronary blood flow abnormalities and consequently myocardial ischemia necessitates complex and time-consuming non-invasive and invasive methods. Widely used non-invasive stress tests have limited diagnostic accuracy for identifying coronary microvascular dysfunction in patients with non-obstructive coronary artery disease and do not rule out coronary microvascular dysfunction in symptomatic patients with non-obstructive coronary artery disease. This underscores the need for invasive assessment or novel more sensitive tests / imaging for these patients. However, interrogation of coronary microvascular pathologies using invasive coronary microvascular indices such as coronary flow reserve (CFR) and microvascular resistance requires thermodilution or Doppler equipped wires that are limited to a few catheterization laboratories with expertise.

Therefore, these cumbersome, indirect, expensive and complex techniques have not gained widespread application for examining coronary microvascular function in this prevalent patient group.

As of today, there is no proven and widely accepted treatment option for this patient group. One of the reasons for this failure in creating an effective treatment option is related to absence of a reliable, easy to use and quantitative method to diagnose microvascular dysfunction. Available methods depend on the detection of impaired perfusion but not aim to identify metabolic and functional deviations of cardiomyocytes in response to impaired reperfusion.

Intracoronary flow

The flow in intracoronary vessels is a function of:

the characteristics of the blood (the fluid),
the characteristics of the vessel (the pipe) at the location being measured/sensed;

the characteristics of the outflow (i.e. the microcirculation); and
the operating characteristics of the heart (the pump).

Blood can only ever move down a vessel as fast as its allowed to leave, i.e. blood can only move as fast as its tightest restriction (whether that be coronary - epicardial, or microvascular).

Fluid flow in the intracoronary environment is difficult to measure. Flow measurements do not reveal the nature of the flow to understand or make a determination of how good or bad the flow is. Practitioners cannot use the flow to make a determination of whether the pressure measurements show a normal flow through a healthy vessel powered by a healthy heart and/or microvasculature, or whether this is an abnormal flow and, if abnormal, what is the root cause or major contributing factor causing the abnormal flow? Is it caused by an unhealthy vessel, by an unhealthy or poorly functioning heart or microvasculature, or is it a combination of these factors? The fluid characteristics of the blood are not an appreciable factor influencing the nature of the flow.

The inability to measure "flow" in this complex and challenging environment is a massive and long-held frustration for practitioners. To get around the inability to measure intracoronary flow, other measurements are taken. The most usual measurement that is taken or sensed is the pressure in the intracoronary vessel. A pressure transducer is mounted at a distal end of a guide wire. Intracoronary measurements of pressure ratios and pressure gradients can be taken. Fractional flow reserve (FFR) and Instant wave-Free Ratio (iFR) deliver FFR and iFR values but these values are derived from pressure measurements. For example FFR is the ratio of pressure distal to a stenosis and pressure proximal to the stenosis taken from the guiding catheter - it is not a measure of actual flow but a pressure ratio.

Doppler analyses can also be undertaken. An ultrasound transducer is mounted at a distal end of a guide wire to transmit ultrasound and receive reflections from blood cells. Blood velocity is calculated using the Doppler principle for that location in the intracoronary vessel. Flow rates can be estimated from this data but intracoronary Doppler traces tend to be used by practitioners. Doppler traces are used to identify peak and mean flow rates. Measurements are made at rest and then after administration of hyperaemic agents such as adenosine. Using a ratio of the resting and hyperaemic flow states, it is possible to gain a measure of the health of the microcirculation, the coronary flow reserve (CFR).

The lack of a "per se" flow measurement hinders practitioners from being able to give an accurate indication of the normalness of the actual flow in the intracoronary vessel where measurements are taken or where images are sensed. Further, practitioners do not have a tool available to them to determine the root cause or major contributing factor to any disturbances or abnormalities in the flow. For example, is the biggest barrier to blood flow an epicardial stenosis or a high outflow resistance in the coronary microcirculation?

Workhorse wires, guide wires and pressure and flow wires

There are two types of coronary wires: "work horse" wires and pressure and flow wires.

1. Work horse wires or guide wires: these are used for all coronary interventions. These do not generally have electrical cables running inside them, and there is sometimes a partial coating to make the wire as slippery as possible to reduce friction between wire and vessel to allow the wire to pass more readily through bends and narrow stenoses. Such wires can be used to carry or contain an electrode. The wire is fed through a catheter to the intracoronary region and is exposed from the catheter. The electrode carried on the wire can be placed in contact with an intracoronary vessel to take ECG readings. There are no conventional or commercial available intracoronary electrocardiographic (ECG) wires - a normal coronary guidewire has been used to carry an electrode. These wires can be steerable and have been adapted to carry one or more electrodes to take ECG measurements. Examples of guide wires are Boston Scientific's CHOICE™ range of wires.

2. Pressure and flow wires: have a pressure sensor and a Doppler flow sensor. Pressure wires can use a piezo-electric (piezoresistive/capacitive/inductive) transducer or sensor to take pressure measurements within a coronary artery. An example is Volcano's PrimeWire range of wires which have a window to a pressure sensor pad located some distance from the distal tip of the wire. An example of a flow wire is the Volcano FlowWire which has a Doppler flow sensor. The Volcano ComboWire has both a pressure sensor and a Doppler flow sensor. A distal end of the ComboWire has a radiopaque distal tip. The radiopaque tip carries both the pressure sensor and a Doppler flow sensor. In one example of the ComboWire the pressure sensor is at the radiopaque tip with the flow sensor and in another example the flow sensor is at the radiopaque tip and the pressure sensor is separated from the flow sensor by the radiopaque tip - 1.5 to 3cm separation. The radiopaque tip is electrically uninsulated. From the distal end of the wire for around 30cm, a SlyDx™ Coating is provided - this is not an electrically insulating coating but is designed to ease friction between the pressure wire and the vessel.

An exemplary intra-coronary wire combining diagnostics and neuro-modulation capabilities is known from document US 2018/116723 A1.

Aspects of embodiments of the present disclosure

The invention is defined in the appended claims.

Embodiments of the disclosure provide practitioners with a new tool useful to help evaluate the intracoronary environment. Embodiments of the disclosure facilitate a new approach and/or technique to evaluate the severity of stenoses and to evaluate coronary microvascular pathologies. Embodiments of the disclosure are particularly useful in the gathering of data and subsequent evaluation of the central microvascular pathology leading to myocardial ischemia and therefore underlying anginal chest pain in patients who do not have flow limiting epicardial coronary artery stenosis. Further, embodiments of the disclosure may also be useful in the detection of the ischemic potential of any given epicardial stenosis.

Embodiments of the disclosure may relate to a system comprising a coronary wire, a method of evaluating the severity of stenosis, identifying the source of ischemia in any given coronary artery supplying myocardial territory; and algorithmic software for evaluating data gathered by systems embodying the invention and identifying even subtle ischemic intracoronary electrocardiographic changes.

One object of embodiments of the present disclosure is to gather data and identify and evaluate functional / structural coronary microvascular pathologies leading to coronary blood flow impairment and consequent myocardial ischemia.

Another object of embodiments of the present disclosure is to interrogate the ischemic potential of any given epicardial stenosis.

A further object of embodiments of the present disclosure is to identify the source of myocardial ischemia (epicardial or microvascular levels in any given coronary artery supplying myocardial territory) when used in combination with pressure sensor.

Yet another object of embodiments of the present disclosure is to eliminate or at least ameliorate the drawbacks outlined in the background of invention.

Other objects of embodiments will become apparent from the accompanying drawings and description.

Description of embodiments

Embodiments of the disclosure will now be described, by way of example, with reference to the accompanying drawings, in which:

Figure 1 is a schematic view of a system, in use, illustrating a distal end of a wire embodiment seen through a cut-away in a vessel wall;
Figures 2 and 3 are schematic representations of prior art pressure and flow wires;
Figures 4 to 7 are schematic views of wire embodiments;
Figure 8 is a schematic view of a wire embodiment located in a catheter;
Figure 9 is a schematic representation of four categories of flows learnt in accordance with embodiments of the present technologies;
Figures 10 to 16 illustrate data sets of: chamber and vessel data being ECG data and/or x-ray imagery; and the associated flow data being pressure readings and Doppler readings;
Figure 17 shows an example of an X-ray of an unobstructed vessel and an X-ray of an obstructed vessel;
Figure 18 shows an example of a normal ECG recording and an abnormal ECG recording;
Figure 19 shows an example of an abnormal: X-ray angiogram, ECG recording and pressure readings;
Figure 20 shows an example of chamber and vessel data indicating a stenosis pre-treatment and the same chamber and vessel data post-stenosis treatment;

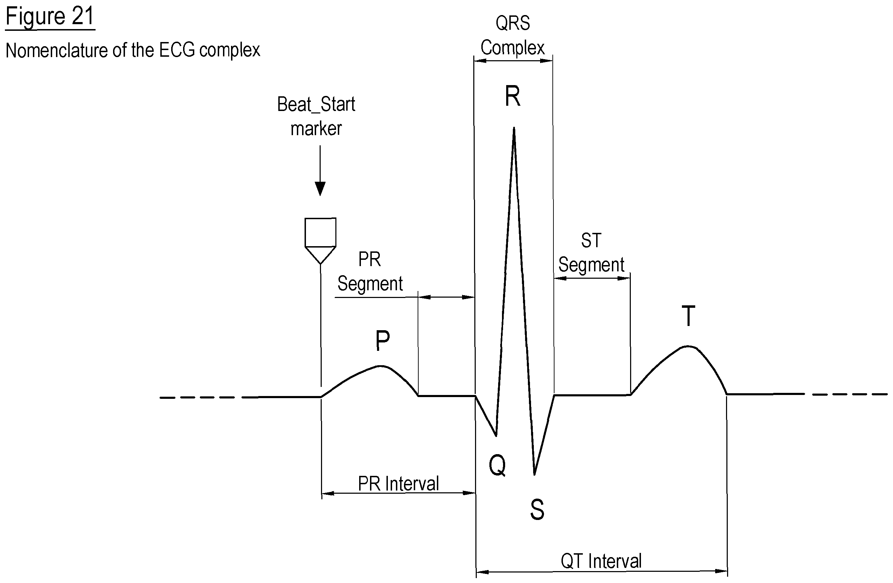

Figure 21 is a schematic representation of a stylised ECG recording of a single beat.

The individual examples, embodiments and illustrations of the present technologies each include individual features. Each of those features or combination of features can be taken from its examples, embodiments and/or illustrations and be applied to any other example, embodiment and/or illustration in any desired combination. The invention is defined by appended claims 1-15.

Embodiments of the present technologies

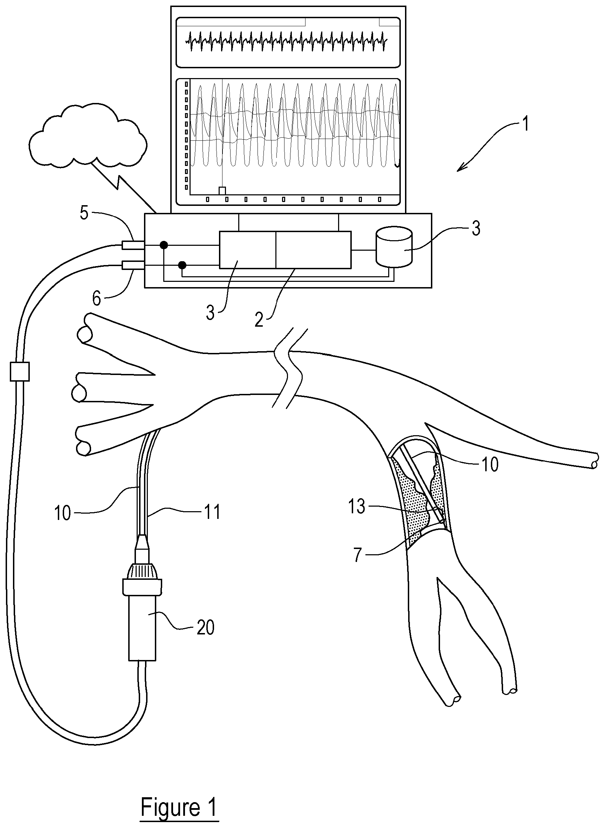

A console or system 1 is shown schematically in Figure 1 and comprises a processor 2 with a signal analyser 3 and data storage 4. The processor 2 has inputs 5,6 which are fed with data signals by one or more intracoronary wires 10 embodying the present invention. The wire 10 is held in a catheter 11 which is inserted in a patient's artery and a distal end of the catheter 11 is guided into the patient's intracoronary region or just short thereof. A portion 12 of the wire 10 is exposed from the catheter 11 and is guided into the intracoronary region to take readings using one or more sensors 7,13 carried at the distal end 14 of the wire 10.

Intracoronary wires

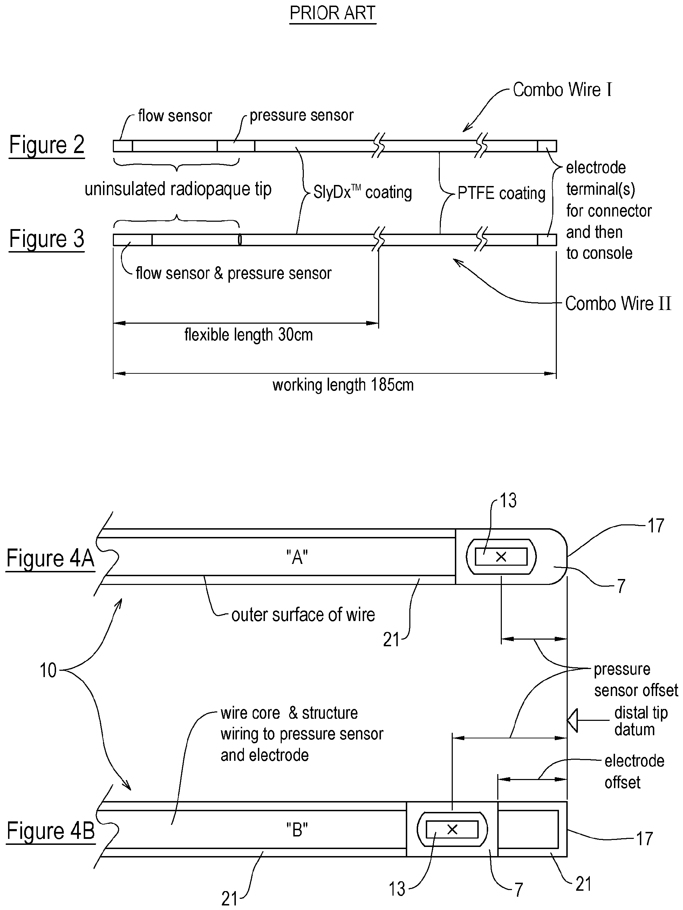

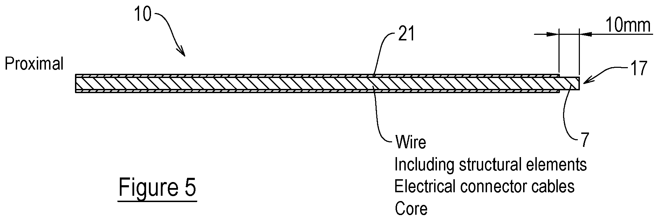

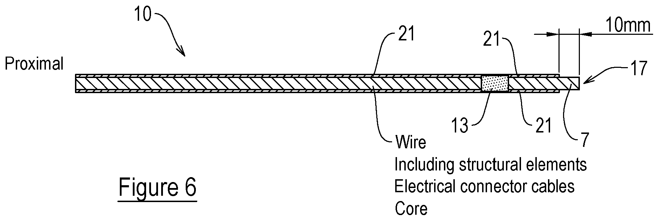

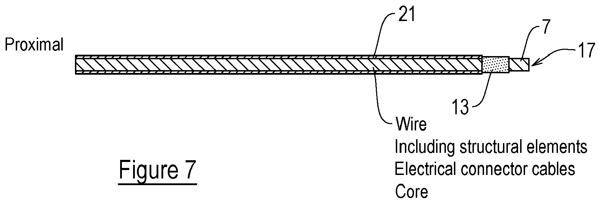

Figures 2 to 8 illustrate various wire embodiments. The features of individual wires can be applied in any combination to features of other wires unless there is an overriding conflict or incompatibility. The invention is defined by the appended claims.

The illustrated coronary wires 10 are wires which comprise or carry one or more leads 15 having electrodes 7 at or near the distal tip 17 of the wire 10. The one or more leads 15 are carried in or on the wire or comprises the wire itself.

The wire 10 may have a structural core or may be empty apart from the one or more leads 15.

A proximal end 18 of the wire 10 exposes electrical contact(s) 19 being the proximal end(s) of the one or more leads 15 in or on the wire. The electrical contact(s) is/are connected to an analyser via a connector 20. The electrical contact(s) 19 of the wire are uninsulated to permit electrical connection to the analyser. Apart from the one or more electrodes 16 or parts of the one or more electrodes, the wire is electrically insulated from the proximal end 18 to the distal end 14 with electrical insulation 21.

Referring to Figures 1, 4, 5, 6 and 7, a coronary wire 10 embodying the present technologies has a central longitudinal axis A with a working length 22 running from a proximal end for connection with the connector to a distal end for insertion through the catheter 11 into a coronary vessel of a patient. Appropriate catheters for this purpose are known. An example of a suitable catheter and wire manipulation/guiding tool is an Extra Backup (EBU), Judkins Left (JL) or Judkins Right (JR) guiding catheter.

When inserted in the patient's intracoronary space, a portion 12 of the wire 10 can be fed out through the distal end of the catheter 11 and is exposed from the catheter - the exposed portion 12 is part of the flexible length 23 of the wire 10. Part of the flexible length 23 of the wire exits the catheter and is able to make contact with the coronary vessel.

| | |
|---|---|
| Catheter 11 length | - typically 90-130cm |
| Working length 22 of wire | - typically 190cm |
| Flexible length 23 of wire | - typically 5-30mm |
| Exposed length 12 of wire exposed from the catheter 11 at the distal | |
| end | - typically 10-100mm |

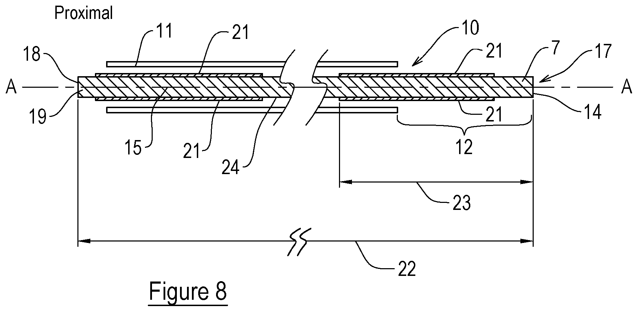

Figure 8 schematically shows a wire 10 in a guiding catheter 11 - for clarity other elements of the guiding catheter such as the torque tool and connector are not shown.

The wire 10 can be any cross-section. The illustrated wire is of generally circular cross-section and adherence to a precisely circular cross-section is not essential. The cross-section can be generally ovoid, generally rectangular or can be modulated having different cross-sections blending in with one another or stepping between cross-sections. Certain cross-sections, particularly towards a distal end of the wire can be useful to steer the coronary wire to its intended destination vessel.

The wire terminates at a distal end 14 in a distal tip 17. The tip 17 can be domed, square or any desired shape to avoid trauma to the vessel.

The diameter of the insulated wire is in the range of 0.3-0.5mm At least one electrode 7 is provided on the outer surface of the working length 23 of the wire which is exposed 12 from the catheter to the intracoronary environment. When the electrode 7 makes contact with the vessel, electrocardiogram signals can be obtained. The electrode 7 may be a single electrode on a unipolar lead 15 or two spaced apart and electrically isolated (from one another) electrodes on a bipolar lead 15. Plural electrodes 7,7' and/or plural leads 15 may be provided and carried on, in or as the wire 10. In the case of a unipolar lead, the electrode 7 is not necessarily a distinct element to the unipolar lead and is just the exposed portion of lead which is uninsulated. The electrode 7 in this case can simply be the end of the unipolar lead or a portion of the unipolar lead exposed to the vessel by virtue of the absence of insulation around the unipolar lead over that electrode length.

The one or more electrodes are provided towards the distal end of the wire. Ideally, the electrode is at or as close to the distal tip as possible. An electrode may be provided at the distal tip of the flexible length or may be offset from the distal tip by a predetermined spacing. The distal tip (0mm offset) provides a datum from which the amount of offset can be set. The offset to the edge of the first electrode can be: in the range of 0mm to 100mm, 5mm to 56mm, 5mm to 36mm, 5mm to 26mm, 5mm to 16mm, 10mm to 16mm.

The length of the electrode along the central axis is in the range of 0.1mm to 30mm, 0.1mm to 20mm, 0.1mm to 10mm, 0.1mm to 5mm, 0.1mm to 3mm, 0.1mm to 2mm, 0.1mm to 1mm, 0.1mm to 0.5mm.

An electrode along a portion of the length of the wire should be sufficiently long to be able to make good contact with the vessel as it lies alongside the vessel, in contrast to a distal tip electrode which can make a good point-to-point contact with the vessel, particularly for a J-shaped tip, and can therefore be "shorter" in the axial direction.

If the wire has two electrodes, the offset to the second electrode is the distance from the distal tip datum to the edge of the second electrode. The offset to the edge of the second electrode can be: in the range of 5mm to 100mm, 5mm to 56mm, 5mm to 36mm, 5mm to 26mm, 5mm to 16mm, 10mm to 16mm, 20mm to 30mm.

Offset Table

| | | Pressure sensor offset | Electrode offset | Electrode length |
|---|---|---|---|---|
| | 4A | 5 mm | 0 mm | 10 mm |
| | 4B | 15 mm | 10 mm | 10 mm |
| | 4C | 25 mm | 0 mm | 10 mm |
| | 4D | 35 mm | 5 mm | 10 mm |
| 1st electrode 7 | 4E | 10 mm | 0 mm | 5 mm |
| 2nd electrode 7' | | | 15 mm | 5 mm |

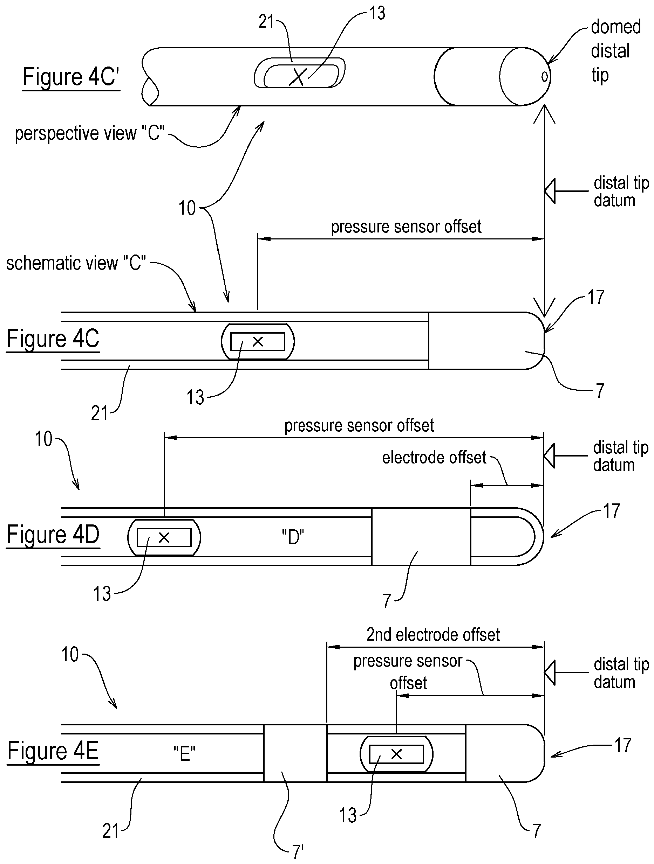

There is substantially no pressure sensor offset as the pressure sensor is located in the electrode - an example of this is Figure 4A. Importantly, there is electrical insulation 21 electrically insulating the exposed section 12 of the wire - the portion of the flexible length 23. For example, the electrical insulation 21 is over the outer surface of the flexible length 23 of the wire. The electrical insulation 21 isolates this length of the wire from the intracoronary environment so that the only place where there is electrical contact between the electrode and the vessel is at the electrode which is the only part of the flexible length of the wire which is not electrically insulated or isolated. The electrical insulation 21 may be over the outer surface of the flexible length 23 except at the electrode at the distal end to allow the electrode to electrically contact the coronary vessel.

In some embodiments, the electrical insulation is a coating on the outer surface of the flexible length. In other embodiments, the electrical insulation is a sleeve over the outer surface of the wire. In some embodiments, the electrical insulation can be a combination of a sleeve and a coating over different portions of the flexible length.

The outer surface of the flexible length of the wire is insulated by either a sleeve, a coating or a combination thereof. The wire itself may be manufactured from an electrically insulating material so that no additional electrical insulation may be required - in this embodiment the electrical insulation is the outer surface of the wire.

Preferably, the electrical insulation 21 is along the flexible length 23 of the wire which is exposed from the catheter to the intracoronary environment. The electrical insulation can be provided along the working length 22 of the wire. Portions 24 of the working length 22 of the wire which remain within the catheter during ECG signal acquisition do not need to be electrically insulated. In such cases, the catheter can be electrically insulating or a coating or sleeve provided on the internal surface of the catheter. Catheter electrical insulation is less important than electrical insulation along the exposed (from the catheter) flexible length as the catheter is less likely to be in a region in which there are strong ECG signals - it is the flexible length of the wire or the part of the flexible length of wire exposed from the catheter which is subject to the strongest intracoronary ECG signals where electrical insulation is targeted.

Previously, coronary wires are not insulated at the distal end and/or along the flexible length of the wire. Dedicated electrical insulation along the flexible length is not designed into conventional coronary wires. Coatings are applied to the flexible length of coronary wires but these are not fully electrically insulative coatings. The main function of these coatings is to reduce friction between the outer surface and the vessels and to allow for ready manipulation, guidance and promotion along the vessels.

The electrical insulation 21 of the exposed portion 12 of the wire 10 except for the electrode(s) 7 is deliberate and effective. The insulation material providing the electrical insulation, be it a coating, a sleeve or the material of the lead or wire itself has a resistivity (in $\Omega$.m) greater than $1\times10^6$ $\Omega$.m and preferably greater than $1\times10^{12}$ $\Omega$.m, $1\times10^{13}$ $\Omega$.m, $1\times10^{14}$ $\Omega$.m.

By insulating all areas of the exposed wire except the electrode(s), the electrical contact between the vessel and the flexible length of the wire is deliberately and effectively constrained to only the electrode(s) so that the point at which ECG data is acquired is the small area of contact between the uninsulated electrode and the vessel with which the electrode is in contact.

The electrode may be a domed tip, a square cap or a flat tip electrode 7. A flat tip electrode is a circular plate with its major face normal to the central axis A. When configured as a tip electrode, the electrode can adopt any shape conducive to establishing a good electrical connection to the vessel.

The electrode may be a plate electrode lying along the wire, an annular electrode at least partially surrounding the wire or a ring electrode encircling the wire.

When taking ECG readings distal of a stenosis, it is important that the location of the readings is known (for example verified by imaging techniques). For example, one location would be a point in the distal area beyond the stenosis. Rather than receiving readings or noise from regions in the stenosis or worse still proximal of the stenosis, there is good confidence with embodiments of the invention that the reading comes from a point distal of the stenosis. If the flexible length of the wire was uninsulated, then there would be opportunity for the ECG readings to be corrupted by input to the electrode from other parts of the wire which might be making better contact with the vessel than the electrode - there are strong ECG signals in the intracoronary region - the electrode could be giving a "creep" reading from another part of the wire as opposed to being constrained to just the electrode itself. Further, the electrical insulation provides a cleaner ECG signal and also an ECG signal which is more clear-cut than for an uninsulated wire and indicative that the electrode is in good electrical contact with the vessel - the ECG signal has a binary status rather than being influenced with ECG noise from other parts of the vessel which are in contact with the wire.

The coronary wires according to the disclosure deliver a clear-cut and location-specific intracoronary ECG signal unaffected by ECG noise generated in other areas of the vessel not in contact with the electrode(s).

In other embodiments of the disclosure, the intracoronary wire further comprises a pressure sensor in addition to the electrode (ECG sensor) toward the distal end of the flexible length. The pressure sensor may be a piezo-electric (piezo-resistive/capacitive/inductive) transducer or an optical-type pressure sensor. The pressure sensor is preferably recessed into the wire so that a pressure pad or surface membrane of the pressure sensor/transducer - the area where pressure is measured is not in direct contact with the vessel. The recessing promotes reception of clear pressure readings and helps minimise the chances of the pressure sensing area being "knocked" and delivering a false pressure reading or artefact.

The pressure sensor can be at the distal tip facing along the length of the wire or can be located along the wire. In some embodiments, the pressure sensor is offset from the distal tip by a predetermined spacing. The pressure sensor offset is measured from the distal tip to the centre of the pressure sensing area of the pressure sensor.

The pressure sensor offset may be around 30mm. The pressure sensor offset is in the range of 0mm to 100mm, 5mm to 56mm, 5mm to 36mm, 5mm to 26mm, 5mm to 16mm, 10mm to 16mm. The pressure sensor offset is measured from the distal tip to the centre of the pressure sensing area of the pressure sensor.

The pressure sensor is located in the electrode so that both sensors are co-located at the distal tip or offset therefrom - Figures 4A and 4B. By co-locating the pressure sensor and the electrode, there is good confidence that the signals received from both sensors are from the same location in the patient's intracoronary vessel.

The recess to the pressure sensing area may be in the form of a window in the electrical insulation which frames the pressure sensing area. Preferably, the pressure sensing area in the window is electrically insulated.

The electrical insulation at the location of the pressure sensing area can be a membrane which is not attached to the pressure sensor and which is deformable to transmit pressure experienced by the membrane to the pressure sensing area.

The illustrated wire in Figure 2 is a pressure wire which has a physical cut-out or window exposing a pressure-sensitive pad - the pressure sensing area - the sensor measures the pressure experienced by the pad (or portion of pad) exposed by the window.

A coronary guide-wire allowing to record intracoronary electrocardiography beside its other functions related to coronary interventions and/or coronary physiology studies, said guide-wire comprising a 0.35-mm (0.014-inch) guide-wire having electrically insulated proximal and distal ends. Between these two ends the whole body of the guide-wire is electrically isolated without any interruption by an appropriate coating. The guide-wire is characterized in that to receive electrocardiographic signals from its distal un-isolated end located in a distal coronary artery which is under investigation, transmits this signal through its isolated body to the un-isolated proximal end, which is connected to a recording and analysing system that is capable of unipolar ECG recording. The guide-wire may have other signal receiver such as pressure sensor and a line conveying data obtained from the pressure sensor.

Embodiments of the present disclosure provide a coronary guide wire for detection of ischemia caused by impaired coronary flow in the myocardial territory supplied by interrogated coronary artery. This guide-wire comprises a 0.35-mm (0.014-inch) metallic wire having an electrically isolated (polymer/plastic, hydrophilic coating) body / shaft and un-isolated proximal and distal ends and stainless steel core. ECG signals from the myocardial territory under examination supplied by interrogated vessel are collected by distal un-isolated (un-insulated) tip of the guide-wire positioned distally in the coronary artery (intracoronary ECG) and these signals are transmitted via central metallic stainless steel core to the proximal end. Electrocardiographic data are transferred from this proximal un-isolated end to digital analyser and unipolar ECG recording is made. The guide wire may comprise a pressure sensor located at the 3 cm proximal to the distal tip such that pressure of the intracoronary blood flow can be monitored in real time. The wire may comprise a pressure sensor line for conveying data obtained from the pressure sensor. In use, the intracoronary wire is inserted through a catheter into an intracoronary vessel. The distal end of the wire is exposed to the intracoronary vessel and the tip can be guided into contact with the vessel.

ECG signals from the electrode are reviewed and monitored in real time on the console display and/or recorded to data storage. The ECG signals can be analysed by an analyser or processor.

If the intracoronary wire includes a pressure sensor, intracoronary ECG signals and intracoronary pressure readings are recorded simultaneously. Since the electrode and the pressure sensor are co-located, the practitioner is aware that the ECG signals and pressure readings are for the same intracoronary location. The ECG signals from the electrode and the pressure readings are reviewed and monitored in real time on the console display and/or recorded to data storage. The ECG signals and pressure readings can be analysed by an analyser or processor.

In embodiments of the present technology, the intracoronary wire may also be provided with an ultrasound flow sensor so that ECG readings, pressure readings and flow velocity and other flow metrics can be sensed by a single wire. Additionally, the sensors are all located at the distal end of the wire so are co-located so the readings are all known to originate from the flow at the same intracoronary location.

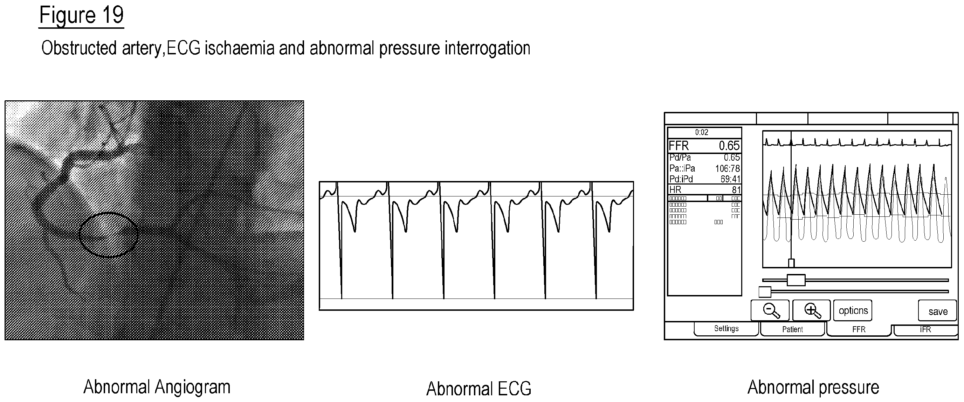

Intracoronary wires embodying the present technology can provide cardiac chamber and vessel data in combination with or separately from flow data (cardiac chamber and vessel data and flow data described below in detail). Figure 19 shows an X-ray of an abnormal angiogram showing a stenosis at "a", the corresponding sensed abnormal ECG measured with an intracoronary wire embodying the present technologies and the corresponding sensed abnormal pressure readings.

Intracoronary data sources

The present technologies sense and collate two distinct types of intracoronary data: chamber and vessel data and flow data.

The term "chamber and vessel data" or "non-flow" data is used to distinguish the chamber and vessel data from the flow data which is/are the pressure waveforms and/or the Doppler waveforms. The data of the data sources is sensed by various mechanisms, including by intracoronary wires described above embodying the present technologies.

Chamber and vessel data sources

In one example, the chamber and vessel data is the ECG readings, waveform and data. The ECG readings record the electrical activity of the heart's chambers and vessels. In another example, the chamber and vessel data is imaging data of the heart's chambers and vessels, including the microcirculation.

Examples of chamber and vessel data sources are:

• ECG data, both pre- and post- stimulation, sensed at a location in the intracoronary vessel

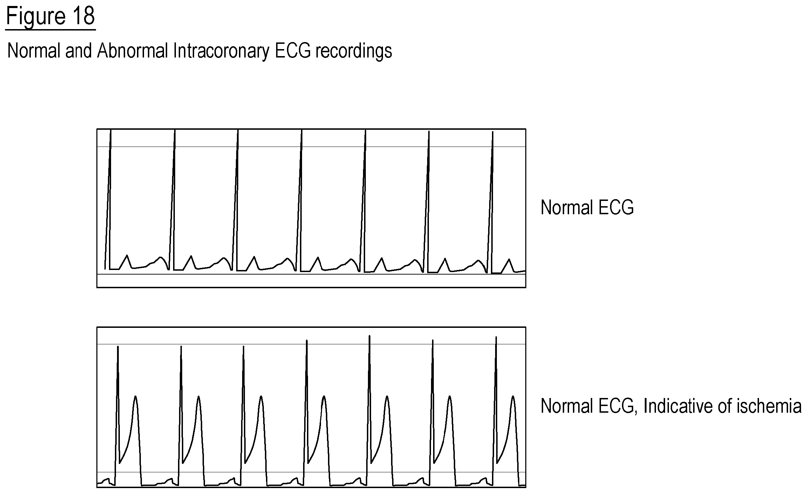

  ◦ see Figure 18 which shows an example of a normal ECG and an abnormal ECG indicative of ischaemia; and

• Imaging data sensed at the location in the intracoronary vessel, both pre- and post- stimulation, including:

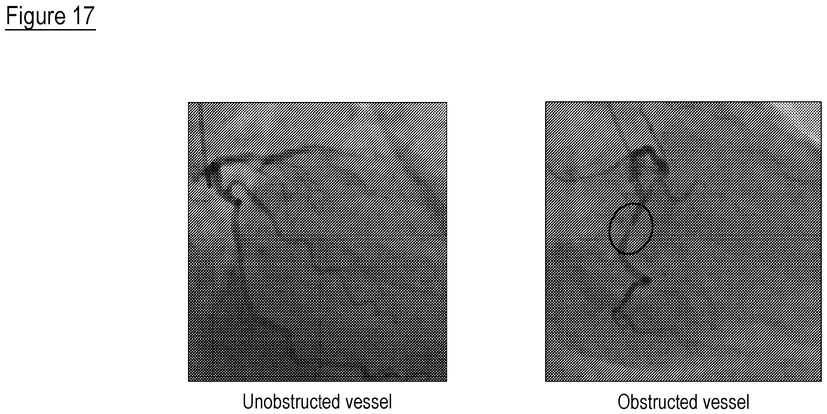

  ◦ X-ray images - see Figure 17 which shows an example of an X-ray of an unobstructed vessel and an X-ray of an obstructed vessel;
  ◦ cardiac magnetic resonance imaging,
  ◦ coronary angiogram
  ◦ Doppler imaging

Flow data sources

The flow data is/are the pressure waveforms obtained from the blood flow and/or the Doppler waveforms taken of the blood flow.

In one example the flow data can be the pressure readings, waveform and data. In another example, the flow data can be the Doppler readings, waveform and data. In a preferred example, the flow data is both pressure data and Doppler data. The Doppler data can be e.g. peak and mean flow rates with measurements made at rest and then after administration of hyperaemic agents such as adenosine and also the entire Doppler waveform.

Taking the specific example of ECG data as the chamber and vessel data and the combination of the pressure waveforms and the Doppler waveforms as the flow data, embodiments of the invention are discussed below.

Other examples of data which can be used as flow data which might contain flow information or vestiges of flow information are:

Tracking the speed of contrast down the length of the vessel on angiography
Using thermodilution techniques
Using other invasive ultrasound-based technique
Using non-invasive ultrasound-based technique
TIMI blush grade

Note: these examples are less-preferred examples of flow data and are non-desirable, non-ideal and potentially impractical compared to flow data sources such as pressure and Doppler data.

Beat identification and beat splitting

Before any training, learning or analysing can be conducted, both the flow data and the cardiac chamber and vessel data is split into beats, each having a beat start - a number of consecutive beats forms a beat array. The beat starts and beat arrays can be aligned so that chamber and vessel data and flow data from the same beat window is being trained against and analysed against one another.

Two neural networks are used to split beats and then classify the cardiac chamber and vessel data (ECG) and flow elements of the beats.

In one example of beat splitting:

- Data is input from ECG and flow traces in 10ms increments.
- This data is split into 3s chunks with a sliding window of 100ms (so 0-3s, 0.1s-3.1 s, 0.2s-3.2s).
- Each chunk is passed into a convolutional neural network of the structure:

  ○ Convolution Layer with 10 filters and a kernel_size of 6
  ○ ReLU non-linearity
  ○ Max Pooling Layer
  ○ Convolution Layer with 10 filters and a kernel_size of 5
  ○ ReLU non-linearity
  ○ Max Pooling Layer
  ○ Convolution Layer with 10 filters and a kernel_size of 4
  ○ ReLU non-linearity
  ○ Max Pooling Layer

- The data is then flatten and passed into a Densely connected network with 300 outputs, each output representing the probability that that point in the network contains a beat-start
- Groups of 10 outputs are then analysed to look for the achievement of a threshold marking the beat start. If a threshold is crossed, the lowest flow point is marked as the start of a beat.
- The source data is then split into an array of beats based on the beat-markers at the start of each beat. Training and/or machine learning: the classification network
- Three sets of beats are grouped, and the ECG and Flow values for them are passed into two networks of identical structure:

  ○ Convolution Layer with 40 filters and a kernel_size of 3
  ○ ReLU non-linearity
  ○ Max Pooling Layer
  ○ Convolution Layer with 30 filters and a kernel_size of 4
  ○ ReLU non-linearity
  ○ Max Pooling Layer
  ○ Convolution Layer with 20 filters and a kernel_size of 5
  ○ ReLU non-linearity
  ○ Max Pooling Layer
  ○ A Bidrectional Long Short Term Memory Layer with 100 units

- The outputs of the two networks are then fed into a densely connected network structured as follows:

  ○ Densely connected layer with 100 units
  ○ ReLU non-linearity
  ○ Densely connected layer with 50 units
  ○ ReLU non-linearity
  ○ Densely connected layer with 1 units

The last layer predicts a value of 0-10 representing the quality of the flow, a flow quotient.

Training the networks:

- All networks are trained using an ADADELTA optimiser to minimise the mean squared error loss function.
- The networks are trained for 20 epochs.

The chamber and vessel data (ECG) is trained against the flow data (pressure and Doppler waveforms).

The training is conducted on the entire dataset not on individual features or characteristics of the data. An experienced practitioner could potentially see characteristics of the ECG waveform and discern that there may be an underlying abnormality in the flow - for example, a deviation from baseline at the ST segments (either depression or elevation) or the duration/QRS width. However, the machine learning does not focus on any particular deviations and instead has a staring-array eye which sees differences in the ECG waveform.

There is a link between the characteristic features of the ECG waveforms and the features of the flow waveforms and data - although humans can make arbitrary calls such as associating low flow with ST segment depression on the ECG, the learned system is not constrained to such human inputs.

The machine learning algorithms do not tie into a particular feature on the ECG and link it to a flow state: it is more sophisticated than that, recognising composites of features that are important. In this manner, the chamber and vessel data (ECG) is trained against the associated flow data (pressure and Doppler waveforms).

The entire ECG waveform is fed into the machine learning algorithms in conjunction with all the flow measurements: the pressure readings and Doppler waveforms. So although the skilled practitioner can detect abnormalities in the ST segment by eye, the system looks at the entire waveform as part of the learning component.

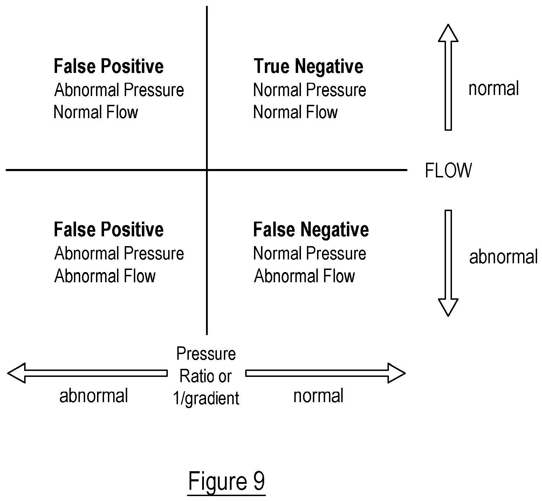

In embodiments of the disclosure, machine learning algorithms are trained to identify characteristic ECG features (chamber and vessel data) against the measured Pressure and Doppler flow signals (flow data) - see Figure 9:

When the flow patterns are representative of **abnormal** flow, the corresponding ECG is trained against that so that the system learns what ECG features are characteristic of an abnormal flow - the machine learning and training exposes the links between the chamber and vessel data and the flow data specifically for abnormal flows.

Similarly, when the flow patterns are representative of **normal** flow, the corresponding ECG is trained against that so that the system learns what ECG features are characteristic of a normal flow - the machine learning and training exposes the links between the chamber and vessel data and the flow data specifically for normal flows.

Similarly, when the flow patterns are representative of **obstructed** flow (a stenosis at the location in the vessel), the corresponding ECG is trained against that so that the system learns what ECG features are characteristic of an obstructed flow - the machine learning and training exposes the links between the chamber and vessel data and the flow data specifically for obstructed flows.

Similarly, when the flow patterns are representative of **unobstructed** flow (lack of stenosis in vessel), the corresponding ECG is trained against that so that the system learns what ECG features are characteristic of an unobstructed flow - the machine learning and training exposes the links between the chamber and vessel data and the flow data specifically for unobstructed flows.

Machine learning was used to categorise a library of data of ECGs having the characteristic features of the following patterns of disease. The system was trained on data representative of the following four groups, see Figure 9:

Unobstructed, normal flow - this is indicative of a healthy epicardial and microvascular bed - normal FFR

Obstructed, normal flow - this is indicative of a disease epicardial artery with no evidence of ischaemia (causes chest pain) - FFR is less than 0.8

Obstructed, abnormal flow - this is indicative of a diseased epicardial artery with evidence of ischaemia - FFR is less than 0.8

Unobstructed, abnormal flow - this is indicative of a microvascular disease pattern, evidence of ischaemia - normal FFR

An unobstructed artery is taken as an artery with no significant stenosis identifiable by imaging, for example, X-ray, and no significant abnormality on the pressure wire in comparison to the reference signal (aortic, or other arterial signal).

An obstructed artery is taken as an artery with significant X-ray stenosis, a significant abnormality on the pressure wire in comparison to the reference signal (aortic, or other arterial signal).

Abnormal flow is derived from characteristic features on the ECG such as when the ECG ST segment or QRS width deviates from the baseline. In essence, machine learning algorithms are trained to identify characteristic ECG features against the measured Doppler flow signal. When flow patterns are abnormal, the corresponding abnormal ECG is trained against it. Similarly when flow patterns are normal, the corresponding normal ECG is trained against it.

Whilst the system has been trained using these four categories or groups to give, for example, assignable flow quotients, it is envisaged that further training can be done using sub-groups defined within each category to give better resolution and "more accurate" flow quotients.

The learnt data set therefore holds technical information linking certain characteristics or features of the chamber and vessel data (ECG) with certain characteristics or features of the flow data (pressure and Doppler).

Analyser

Embodiments of the present technology comprise a console 1 being a system 1 having local, remote and/or distributed components. In this example the components are all local housed in the console 1. The analyser 3 includes an interface and/or algorithm software for receiving and processing real time sensed intracoronary ECG data from the distal un-insulated distal end of a wire and sensed real-time flow data. The data can be displayed on a screen as shown schematically in Figure 1. The sensed data can be stored in data storage 4 in the console 1 or remote therefrom. The learnt data set may be available to the processor 2 and analyser 3 for analysis against the sensed data. The analysis can be performed in real-time or may be conducted off line, in batches or delayed by a predetermined time.

The learnt data set holds technical information linking certain characteristics or features of the chamber and vessel data (ECG data) with certain characteristics or features of the flow data.

The analyser 3, discussed below, receives:

sensed chamber and vessel data only;
sensed flow data only; or preferably
sensed chamber and vessel data in combination with sensed flow data from a patient.

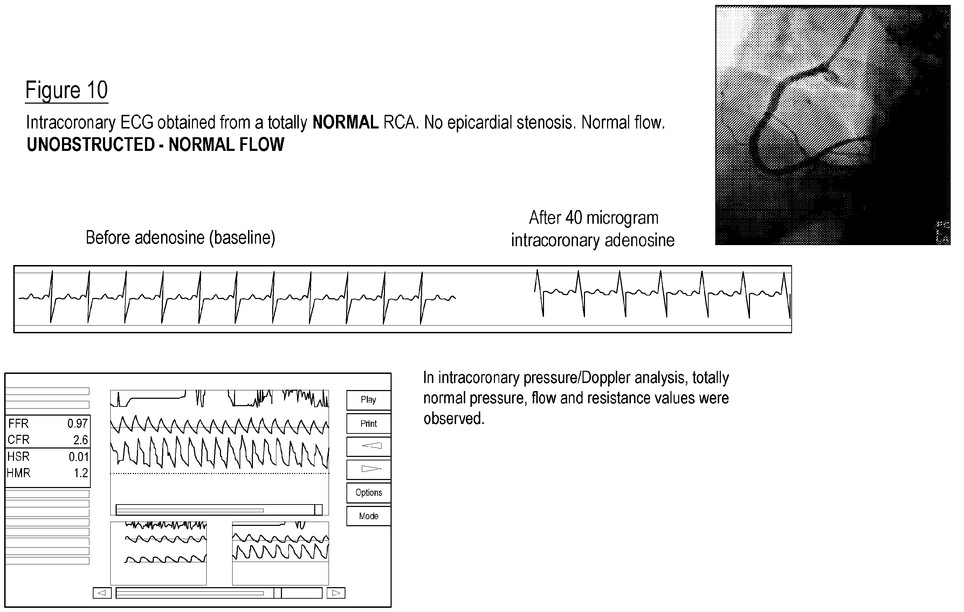

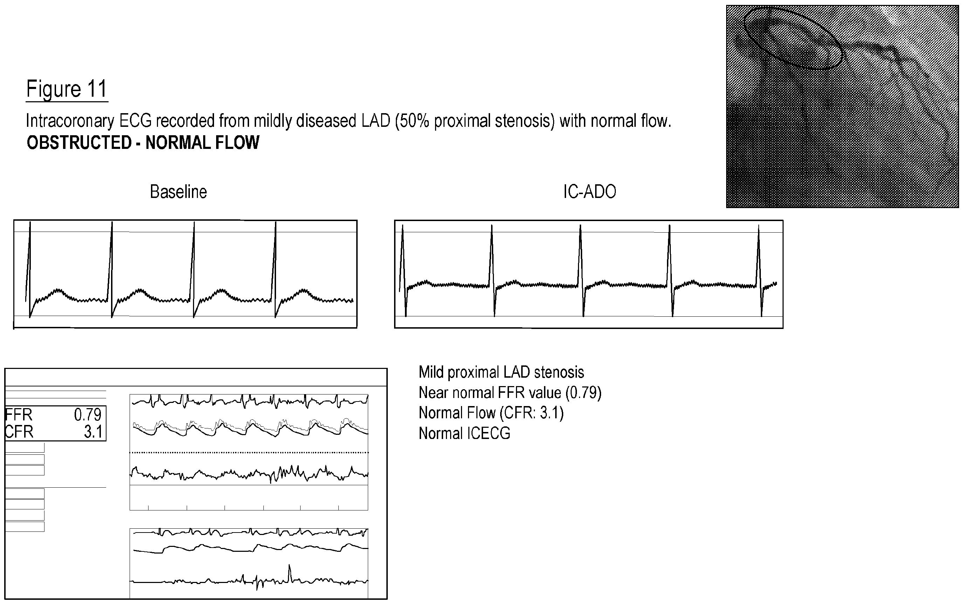

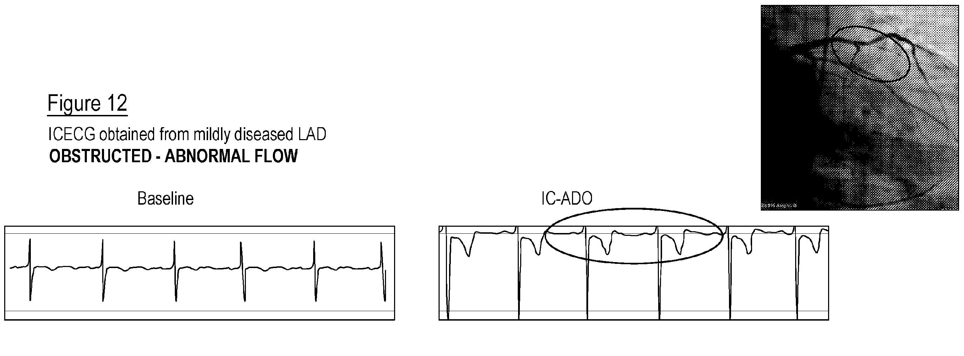

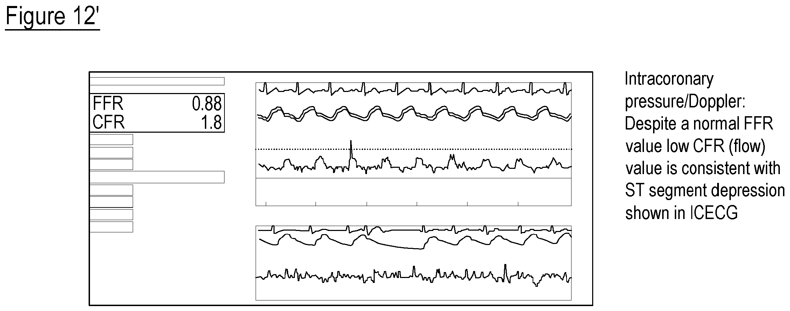

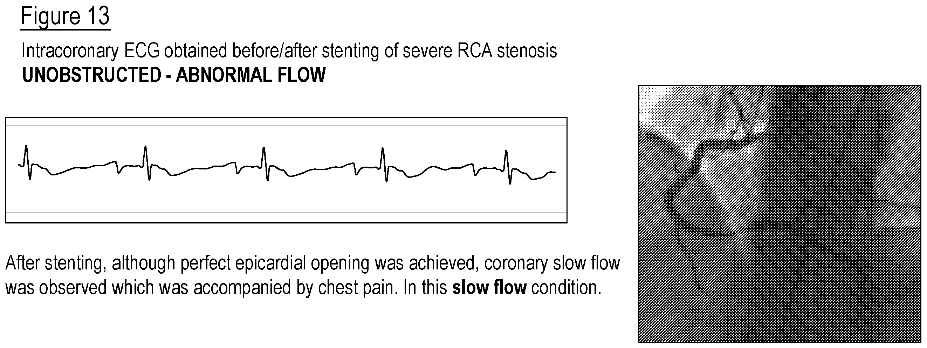

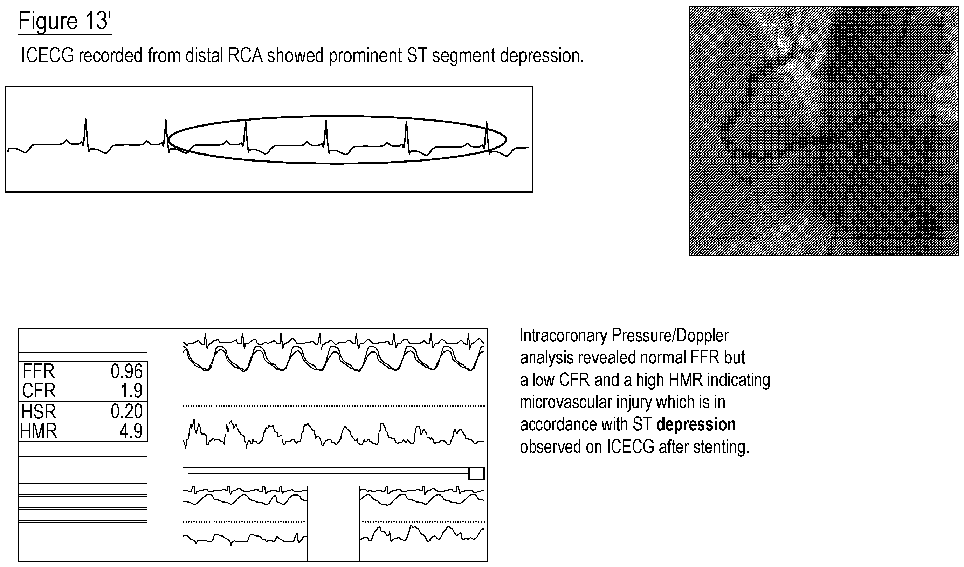

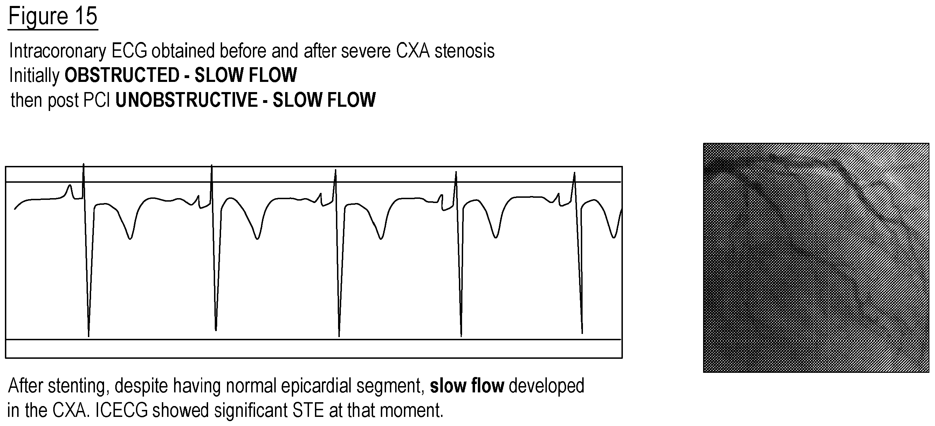

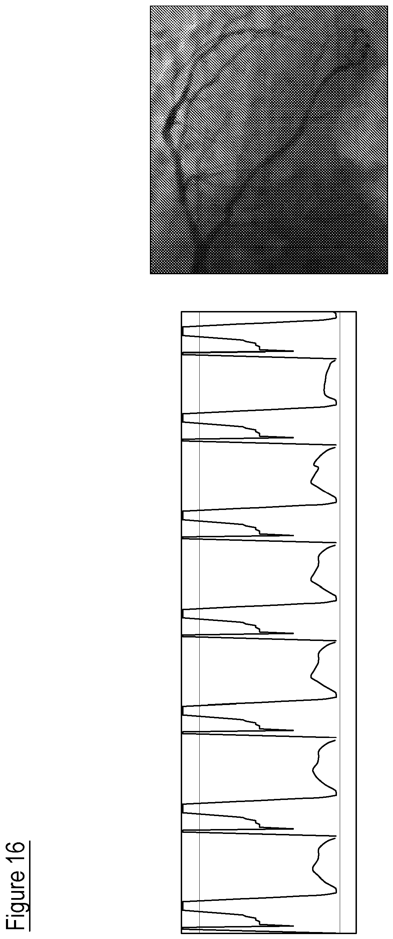

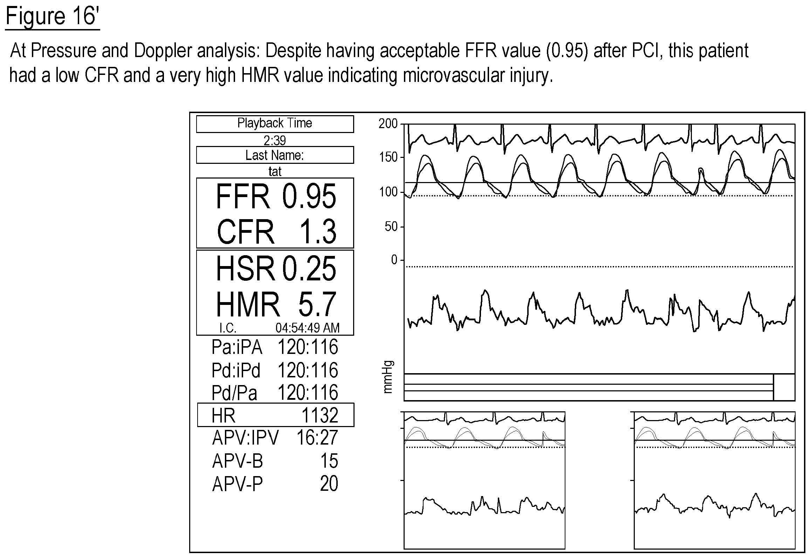

The analyser 3 interrogates the learnt data set to categorise the sensed data as relating to a normal/abnormal flow and/or an obstructed/unobstructed flow. Figure 9 is a schematic representation of the four quadrants into which the flow can be categorised. Figures 10 to 16 illustrate six distinct data sets of: chamber and vessel data being ECG data and/or x-ray imagery; and the associated flow data being pressure readings and Doppler readings. These data sets are characterised as follows and can be used in the data set for training and as part of the learnt data set for analysis against sensed data:

| Figure | Obstructed/ unobstructed | Normal/ abnormal Flow |
|---|---|---|
| 10 | Unobstructed | Normal |
| 11 | Obstructed | Normal |
| 12 | Obstructed | Abnormal |
| 13 | Unobstructed | Abnormal |
| 14 | Unobstructed | Normal |

(continued)

| Figure | Obstructed/ unobstructed | Normal/ abnormal Flow |
|---|---|---|
| 15 | Obstructed | Abnormal |
| 16 | Unobstructed | Abnormal |

This proposed coronary guide wire equipped with intracoronary ECG and intracoronary pressure recording capabilities will enable identification of the source of the myocardial ischemia. Assessment may be performed at rest, or after induced hyperemic (adenosine, papaverine, dipyridamole, nitroprusside, etc.) or vasoactive (acetylcholine) substances may induce significant ST segment shift that can be highly accurately detected by this proposed guide wire and its software. In the absence of the significant epicardial stenosis (angiographically or hemodynamically defined), this finding identifies microvascular disease as the source of myocardial ischemia. Therefore, this proposed guide wire provides electrocardiographic evidence of ischemia in the myocardial territory supplied by normal / near normal epicardial coronary arteries which indicates the presence of microvascular disease/dysfunction.

Using embodiments of the disclosure can present a practitioner with evaluation material from which to make a straightforward and accurate diagnosis of microvascular dysfunction.

The analyser takes inputs from sensed flow-based information and analyses that against a learnt data set. The learnt data set and its derivation is discussed above. The result of the analysis is one or more of:

a determination of the category of the flow being sensed - whether that flow is obstructed or unobstructed and normal or abnormal;
delivery of a derived flow quotient indicative of the nature of the flow being sensed;
a determination of the severity of a stenosis in the flow being sensed; and/or
a positive determination minimising the occurrence of false positives and false negatives - see Figure 9.

For example, using the intracoronary wire of the present technologies and the analyser (with learnt data set of the present technologies) allows an evaluation of the intracoronary flow which assists in decision-making where there is a discordance between the FFR and CFR readings. The present technologies provide additional flow information useful as an evaluation tool.

Using the present technologies, practitioners can identify the presence of microvascular angina in patients with otherwise normal FFR values but having non-flow limiting stenosis.

Flow quotient

This specification refers to a flow quotient. The flow quotient denotes the presence, degree and quality of fluid flow at a location in an intracoronary region in a patient's body, for example in an intracoronary artery. In the context of the present technology, it is important to note that the flow quotient is a derived parameter or characteristic related to the flow but surprisingly, the flow quotient is not derived from flow data such as the flow velocity or the pressure of the flow but is derived from cardiac chamber and vessel data such as ECG data sensing the electrical activity of a chamber or vessel and imaging data sensing the relative size of any stenoses, for example, in an X-ray image of a chamber and/or vessel.

Whilst not necessarily a value, as such, the flow quotient is an indicator of the normality or otherwise of arterial blood flow in an intracoronary vessel. The flow in the vessel is a function of:

the characteristics of the blood (the fluid),
the characteristics of the vessel (the pipe) at the location being measured/sensed;
the characteristics of the outflow (i.e. the microcirculation); and
the operating characteristics of the heart (the pump).

The flow quotient may be a flow quotient weighting factor not an actual flow quotient value.

The flow quotient is a measure of "normalness" of the flow and is high for normal blood, normal vessel and normal heart operation. The flow quotient is low if there abnormalities in any or all of blood, vessel or heart.

For a normal vessel which is free from disease, furring or stenosis has a "good" flow and contributes to a high flow quotient, for example, 10/10. In contrast, an obstructed, poor or diseased intracoronary vessel affects the flow which may be decreased, interrupted, irregular or spasmodic and therefore contributes to a low flow quotient, for example 1/10.

For a normal heart which is free from disease and operates normally creates a "good" flow in the intracoronary vessels and contributes to a high flow quotient, for example, 10/10. In contrast, a diseased, or severely diseased microcirculation creates a "poor" flow which may be decreased, interrupted, irregular or spasmodic - and contributes to a low flow quotient, for example 1/10.

The flow quotient for a particular location in an intracoronary vessel is not measurable per se. Fluid flow in the intracoronary environment is difficult to measure but it is the very measure that medical practitioners wish to evaluate - the inability to measure "flow" in this environment is a long-held frustration for practitioners. To get around the inability to measure intracoronary flow, other measurements are taken and other characteristics of the flow are sensed. The most usual measurement that is taken or sensed is the pressure in the intracoronary vessel. Measurements of pressure ratios and pressure gradients can be taken. Fractional flow reserve (FFR) and Instant wave-Free Ratio (iFR) deliver FFR and iFR values but these values are derived from pressure measurements. For example FFR is the ratio of pressure distal to a stenosis and pressure proximal to the stenosis - it is not a measure of actual flow but a pressure ratio.

The flow quotient gives an accurate indication of the normalness of the actual flow in the intracoronary vessel where measurements are taken or where images are sensed depending on the embodiment of the invention. The flow quotient is derived from one or more data sources but those data sources are not measuring flow, as such. This specification refers to these data sources as chamber and vessel data sources. A processor derives the flow quotient which contains local intracoronary flow information - the derived flow information is obtained from intracoronary "chamber and vessel" data.

Embodiments of the system can utilise a dichotomous classification for the flow quotient:

categories 1 & 2 - the "normal flow" ranges between a high flow quotient of 10 down to 6.
categories 3 & 4 have abnormal flows and therefore have lower flow-quotients ranging from 5 to 1.

In other embodiments, the flow quotient is assigned to respective types of flow - e.g:

Normal flow (8-10)
Mild Abnormal Flow (6-7)
Moderate abnormal flow (3-5) and
Severe abnormal flow (1-2).

The learned data set can be interrogated (and supplemented) with further data to further the learning process. The analyser 3 is discussed in further detail below but, in short, the analyser receives sensed chamber and vessel data (ECG) and sensed flow data (pressure and Doppler) from a patient and categorises the sensed data as relating to a normal/abnormal flow and/or an obstructed/unobstructed flow.

With respect to the flow quotient, information about the nature of the flow is derived from "non-flow" data sets of ECGs. The respective associated pressure and Doppler flow patterns for each piece of "non-flow data" e.g. an ECG are analysed, for example. When the pressure and Doppler flow patterns are NORMAL flow patterns, then the associated ECG pattern is considered by the system to represent normal flow and therefore flow with a high flow quotient. Similarly, when the pressure and Doppler flow patterns are ABNORMAL flow patterns, then the associated ECG pattern is considered by the system to represent abnormal flow and therefore a low flow quotient. In this manner, flow quotients are assigned to recognised characteristics in the ECG pattern.

Further, the learnt data can also be used to allocate flow quotients to analysed chamber and vessel data such as ECGs and imaging data. For example, the allocation of a flow quotient to a particular data set can be used to assess the severity of an arterial stenosis at the sensed location using the below methodology:

Mathematical derivation and solution for incorporation of flow-quotient as a substitute for measured flow for the evaluation of coronary artery stenosis

$$\text{Equation 1.} \qquad \frac{\text{Pressure ratio or gradient}}{\text{Flow}} = \text{Stenosis severity accounting for microcirculation}$$

But when flow is unknow, pressure ratio or gradient can either be under or overestimated

But, by using machine learning, it is possible to train ECG to approximate flow. So in this case,

$$\text{Equation 2.} \qquad \text{Flow-quotient} \propto \text{Trained ECG}$$

Now, Equation 1 can be re-written as,

$$\text{Equation 3.} \qquad \frac{\text{Pressure ratio or gradient}}{\text{Flow-quotient}} = \text{Stenosis severity accounting for microcirculation}$$

Which resolves the need for direct measurement of coronary flow

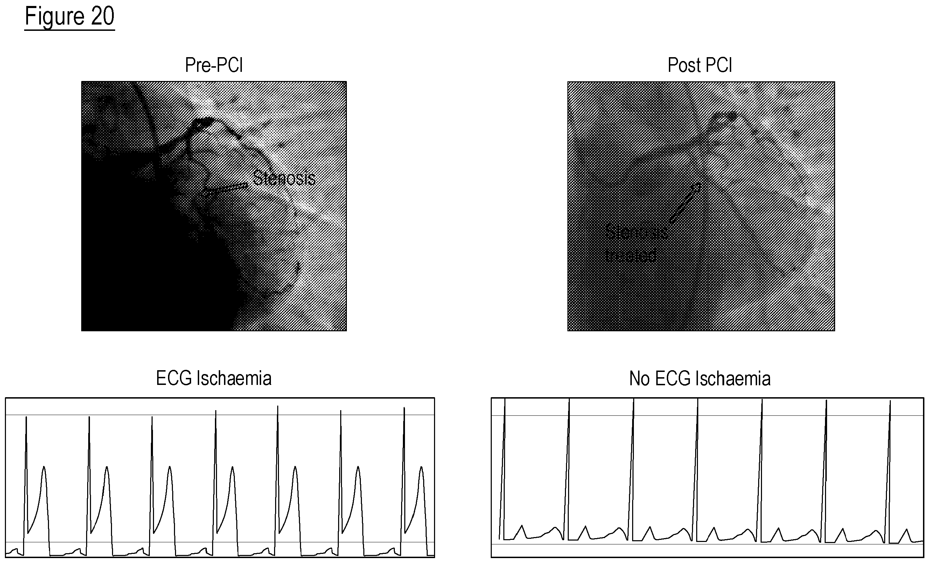

If the chamber and vessel data is imaging data like X-ray imaging data about the chambers and/or vessels, then this can also be trained and learnt with the flow data so that links are established in the learnt data set between the images and the flow data. For example, images such as these can be trained on where (as in Figure 20) the chamber and vessel data (X-ray image) shows an obstructed vessel with a stenosis (pre-treatment) and the same chamber and vessel data (X-ray image) shows the unobstructed vessel post-stenosis treatment.

**It is important to appreciate that embodiments of the present technologies derive flow-related information from non-flow data and create a learnt data set therefrom. That learnt data set can then be used to analyse sensed non-flow data (the chamber and vessel data) and derive information about the nature of the flow from that sensed non-flow data.**

Intracoronary location

The sensed intracoronary data when collected using a wire embodying the present technologies delivers intracoronary data taken from the same intracoronary location in a patient's body. Historical or legacy data which has been gathered using means other than wires embodying the present technologies which can co-locate sensors to the same intracoronary location. Surprisingly, the characteristic links developed in the learnt data set (including co-located and non-co-located data) between the chamber and vessel data and the flow data remain good despite the data set having data acquired without using co-located sensors.

In wires embodying the present technologies, the pressure sensor and the electrode can be co-located so that the practitioner has good confidence that the signals received from both sensors are from the same location in the patient's intracoronary region.

Applications

An angiographically mild (50-60%) LAD stenosis where both of FFR and iFR values (0.78 and 0.85) indicated that the lesion needs to be re-vascularised: correspondingly, the intracoronary wire embodying the present technology (IC-ECG - intracoronary wire with learnt data set) provides a recording yielding a positive result (significant ST depression) in this very mild lesion. This observation implies that the IC-ECG recording under adenosine hyperemia maybe an alternative and sensitive method to detect ischemia even in angiographically mild lesions.

Use of these IC-ECG technologies can dispense with the need to take recordings with stimulant provocation. For example, in the case of a very severe stenosis with angiographical appearance, the IC-ECG technologies recorded from a location distal of the lesion with amplitudes of 5 and 10 mm/mV, showed prominent ST depressions. Correspondingly, Pd/Pa (without adenosine) was very low (0.65). Myocardial ischemia generated by this severe stenosis in the subtended myocardial territory is detected in the resting conditions by the IC-ECG technologies recording without the need for a stimulant (adenosine) provocation - this may also imply the ischemia specificity of the method. After removal of the epicardial stenosis completely, the IC-ECG technologies kept showing prominent ST segment depression and indicating ongoing myocardial ischemia in the RCA territory. At that time, the patient was suffering from chest pain and the angiogram was showing coronary slow flow and FFR value was 0.97. However the IC-ECG technologies were showing prominent ST-segment deviation. These findings imply that the ST depression seen on the IC-ECG technologies indicates myocardial ischemia due to acute microvascular dysfunction reflected by coronary slow flow developed after stenting.

In this particular case, despite having perfect FFR value indicating successful removal of epicardial resistance, ST segment depression on the IC-ECG technologies indicates myocardial ischemia. Therefore, the IC-ECG technologies can also be used to evaluate microvascular status in different disease settings.

Combined use of FFR and the IC-ECG technologies in a patient with chest pain but mild or no stenosis as in cardiac syndrome X, diabetes and other microvascular disease settings, may help to reveal, quantitate and to identify the source of the myocardial ischemia.

The IC-ECG technologies in conjunction with FFR can help us identify the source of the ischemia (epicardial lesion and/or microcirculation). For example, we may have a patient with chest pain and only mild CAD at angiogram. In this particular patient, (-) FFR and (+) IC-ECG may indicate microvascular disease causing myocardial ischemia (-FFR is FFR>0.80. (FFR 1 indicates no difference in ratio between aortic and pressure wire pressures (good), whereas FFR 0.01 (really bad).

For example, using the intracoronary wire of the present technologies and the analyser (with learnt data set of the present technologies = IC-ECG) allows an evaluation of the intracoronary flow which assists in decision-making where there is a discordance between the FFR and CFR readings. The present technologies provide additional flow information useful as an evaluation tool.

Using the present technologies, practitioners can identify the presence of microvascular angina in patients with otherwise normal FFR values but having non-flow limiting stenosis.

Microvascular dysfunction/ spasm causing microvascular angina is diagnosed by assessment of coronary blood flow increase in response to acetylcholine. This is a cumbersome technique requiring intracoronary flow assessment. However, the present IC-ECG technologies can be very helpful in this situation. Intracoronary Ach produces an ST segment shift in this patient subset which can be easily detected by present IC-ECG technologies.

Conventional pressure-based measurements and ECG measurements may indicate slow flow and suggest a stenosis but if cardiac tissue is dead, or non-viable, then in such circumstances, treatment of a coronary stenosis, even in a physiologically positive stenosis (i.e. FFR <0.80) would likely be futile. Opening a stenosis in these circumstances would have no effect on dead heart muscle. Dead or non-viable tissue provides specific ECG characteristic features measurable with (and determinable from) the present IC-ECG technologies - for example, showing "Q" waves on the ECG. As such, incorporation of these specific ECG characteristics provides another level of refinement and determination of the suitability for stenosis treatment.

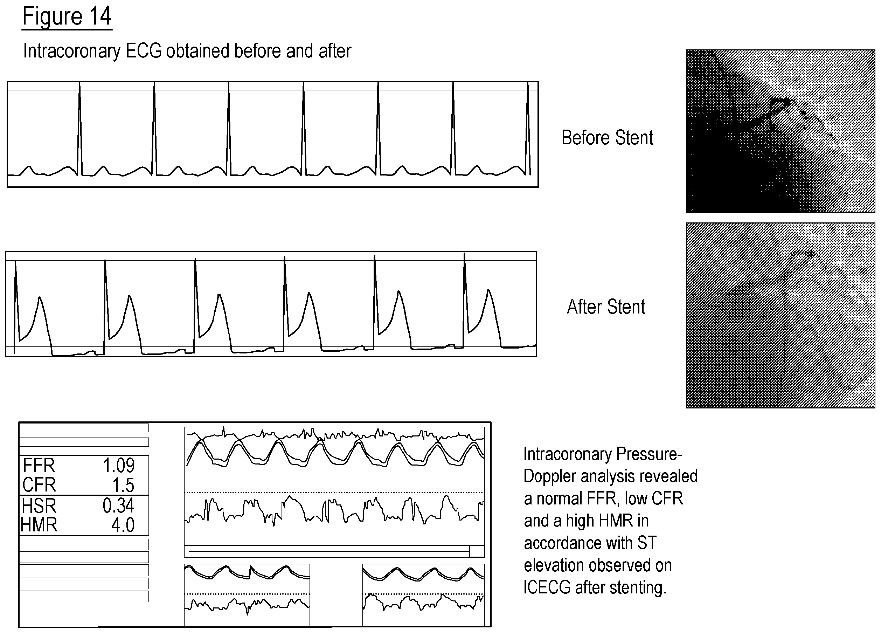

Further, the versatility of the evaluation system embodying the present technologies provides a tool useful for, *inter alia,* pre-post PCI analysis. The analyser delivers both a pre-PCI assessment of the flow, and also an intra-procedural and post-procedural PCI monitoring tool. When there is disruption to the microcirculation post PCI (stenting), then the analyser indicates that there are new changes detected in ECG/flow (see example 4, example 5 and 6 in Figures 14, 15 and 16 respectively). This provides an intra-procedure monitoring tool for detection of ischaemia, and confirmation of success or otherwise of the PCI procedure - i.e. the stenting may look good on the angiogram but the analyser delivers a real picture of what is happening to the microcirculation and blood flow.

The analyser offers the ability to analyse X-ray images and identify how tight a lesion is by an analysis of comparative attribution of flow quotients. The analyser offers the ability to analyse sensed ECGs against a previously measured and trained data set to deliver information about the flow from the ECG alone - the flow quotient being derived from the non-flow ECG data. Further, the analyser offers the ability to analyse sensed non-flow ECG data with sensed pressure based readings (FFR/iFR/PdPa) to derive further flow information.

With these different patterns of disease the information is fed back to the operators. In disease patterns 1, 2 and 4 (examples in Figures 10, 11 & 12 respectively) the operator is advised against revascularization of the artery, which is likely to be futile. In disease pattern 3, the operator is advised to treat the artery with a stent which is likely going to benefit the patient.

When used in this specification and claims, the terms "comprises" and "comprising" and variations thereof mean that the specified features, steps or integers are included. The terms are not to be interpreted to exclude the presence of other features, steps or components.

The invention is defined by appended claims 1-15.

## Claims

1. A coronary wire (10) for insertion through a catheter (11) into a coronary vessel of a patient, the wire having:

   a working length (22) running from a proximal end (18) for connection with a connector to a distal end (14) for insertion through a catheter into a coronary vessel of a patient;
   a flexible length (23), a portion of which is exposed (12), in use, from the catheter and able, in use, to make contact with the coronary vessel;
   a distal tip (17) at the distal end;
   an electrode (7) on the flexible length at the distal tip or offset from the distal tip by a predetermined spacing;
   a pressure sensor (13) located in the electrode; and
   electrical insulation (21) electrically insulating the exposed portion of the flexible length of the wire except at the electrode to allow the electrode to electrically contact the coronary vessel.

2. The wire of claim 1 in combination with the catheter.

3. The wire of any preceding claim, wherein:

   the electrode is offset from the distal tip by a predetermined spacing in the range of 0mm to 100mm, 5mm to 56mm, 5mm to 36mm, 5mm to 26mm, 5mm to 16mm, 10mm to 16mm; and/or.
the wire has multiple electrodes; and/or
the electrode is one or a combination of: a domed tip electrode, a flat tip electrode, a plate electrode, an annular electrode at least partially surrounding the wire and/or a ring electrode encircling the wire.

4. The wire of any preceding claim, wherein the electrical insulation is a coating on the wire or a sleeve over the wire.

5. The wire of any preceding claim, wherein there is a window in the electrical insulation to accommodate a part of the pressure sensor and the part of the pressure sensor in the window is electrically insulated; or
wherein there is a window in the electrical insulation to accommodate a part of the pressure sensor and the part of the pressure sensor in the window is electrically insulated, wherein there is electrical insulation in the window comprising a membrane covering the pressure sensor which is deformable to transmit pressure experienced by the membrane to the pressure sensor.

6. The wire of any preceding claim, wherein the wire has a generally circular cross-section and the diameter of the insulated wire is in the range of 0.35mm to 1.27mm, 0.3mm to 1.5mm, 0.2 to 2mm; and/or
the wire has a central axis and the length of the electrode along the central axis is in the range of 0.01mm to 30mm, 0.1mm to 20mm, 0.1mm to 10mm, 0.1mm to 5mm, 0.1mm to 3mm, 0.1mm to 2mm, 0.1mm to 1mm, 0.1mm to 0.5mm.

7. An intracoronary flow evaluation system (1) comprising:

the coronary wire of any of claims 1 to 6, to provide local intracoronary chamber and vessel data from an intracoronary location in a patient's body;
a learnt data set comprising: intracoronary chamber and vessel data trained against intracoronary flow data;
a first input (5,6) to receive the data from the coronary wire; and
a processor (2) configured to analyse the input intracoronary chamber and vessel data against the learnt data set to derive flow information for the intracoronary location in the patient's body, the derived flow information comprising a tool to evaluate the intracoronary flow at the intracoronary location in the patient's body.

8. The system of claim 7, wherein the intracoronary chamber and vessel data is one or more of:

ECG data of the electrical activity of an intracoronary chamber and/or vessel; and
imaging data of an intracoronary chamber and/or vessel.

9. The system of claim 7 or 8, wherein the intracoronary flow data is one or more of:

intracoronary pressure data of the flow in an intracoronary chamber or vessel; and
intracoronary Doppler data of the flow in an intracoronary chamber or vessel.

10. The system of any of claims 7 to 9, wherein the system has a second input to receive local intracoronary flow data from the intracoronary location in a patient's body and the processor is configured to analyse the input flow data with the input intracoronary chamber and vessel data against the learnt data set and derive flow information for the intracoronary location in the patient's body.

11. The system of any of claims 7 to 10, wherein the system is configured to evaluate a coronary artery stenosis, the processor being configured to derive a flow quotient containing local intracoronary flow information for the intracoronary location in the patient's body and to calculate a pressure ratio or gradient for the intracoronary location in the patient's body from the intracoronary flow data from the second input,
wherein the severity of the stenosis is correlated with a ratio between the calculated pressure ratio or gradient and the derived flow quotient.

12. The system of claim 11, wherein the processor is configured to calculate the severity of the stenosis as: either the pressure ratio divided by the flow quotient, or the pressure gradient divided by the flow quotient.

13. The system of any of claims 7 to 12, wherein the processor is configured to derive a flow quotient containing local intracoronary flow information for the intracoronary location in the patient's body from the local intracoronary chamber and vessel data for the intracoronary location.

14. The system of any one of claims 11 to 13, wherein the processor is configured to derive the flow quotient or range of flow quotients by:

    categorising the learnt data into one or more categories, each category having an associated flow quotient or range of flow quotients; and
    fitting the intracoronary chamber and vessel data to learnt data in a category; and
    allocating the flow quotient associated with that category to the local intracoronary chamber and vessel data from the intracoronary location in the patient's body.

15. The system of any of claims 7 to 14, wherein the data is in the form of a waveform and the learnt data set is trained on the waveform in its entirety or parts thereof and the inputs to the analyser are fed sensed data in the form of a waveform such that analysis takes place based on the waveforms.

**Patentansprüche**

1. Koronardraht (10) zur Einführung durch einen Katheter (11) in ein Herzkranzgefäß eines Patienten, wobei der Draht aufweist:

    Eine Nutzlänge (22), die längsbezogen von einem proximalen Ende (18) zur Verbindung mit einem Verbinder zu einem distalen Ende (14) zur Einführung durch einen Katheter in ein Herzkranzgefäß eines Patienten verläuft;
    eine flexible Länge (23), wovon ein Abschnitt (12), im Gebrauch, vom Katheter exponiert und fähig ist, im Gebrauch, Kontakt mit dem Herzkranzgefäß zu machen;
    eine distale Spitze (17) mit der Spitze am distalen Ende;
    eine Elektrode (7) auf der flexiblen Länge an der distalen Spitze oder um einen vorbestimmten Abstand von der distalen Spitze versetzt;
    einen in der Elektrode befindlichen Drucksensor (13); und
    elektrische Isolierung (21), wobei die Isolierung den exponierten Abschnitt der flexiblen Länge des Drahtes, außer an der Elektrode, isoliert, um der Elektrode zu ermöglichen, das Herzkranzgefäß elektrisch zu kontaktieren.

2. Draht nach Anspruch 1 in Kombination mit dem Katheter.

3. Draht nach irgendeinem vorhergehenden Anspruch, wobei:

    Die Elektrode von der distalen Spitze um einen vorbestimmten Abstand im Bereich von 0 mm bis 100 mm, 5 mm bis 56 mm, 5 mm bis 36 mm, 5 mm bis 26 mm, 5 mm bis 16 mm, 10 mm bis 16 mm versetzt ist; und/oder der Draht mehrfache Elektroden aufweist; und/oder
    die Elektrode ist eine oder eine Kombination von: einer Elektrode mit kuppelförmiger Spitze, einer Elektrode mit flacher Spitze, einer Plattenelektrode, einer Ringelektrode, welche den Draht wenigstens teilweise umgibt und/oder einer Ringelektrode, die den Draht umschließt.

4. Draht nach irgendeinem vorhergehenden Anspruch, wobei die elektrische Isolierung eine Beschichtung auf dem Draht oder eine Tülle über dem Draht ist.

5. Draht nach irgendeinem vorhergehenden Anspruch, wobei in der elektrischen Isolierung ein Fenster vorhanden ist, um einen Teil des Drucksensors aufzunehmen, und der Teil des Drucksensors im Fenster elektrisch isoliert ist; oder wobei in der elektrischen Isolierung ein Fenster vorhanden ist, um einen Teil des Drucksensors aufzunehmen und der Teil des Drucksensors im Fenster elektrisch isoliert ist, wobei elektrische Isolierung im Fenster vorhanden ist, die eine den Drucksensor bedeckende Membran umfasst, die verformbar ist, um von der Membran erfahrenen Druck auf den Drucksensor zu übertragen.

6. Draht nach irgendeinem vorhergehenden Anspruch, wobei der Draht einen generell runden Querschnitt aufweist und der Durchmesser des isolierten Drahtes im Bereich von 0,35 mm bis 1,27 mm, 0,3 mm bis 1,5 mm, 0,2 bis 2 mm liegt; und/oder
der Draht eine mittige Achse aufweist und die Länge der Elektrode im Bereich von 0,01 mm bis 30 mm, 0,1 mm bis 20 mm, 0,1 mm bis 10 mm, 0,1 mm bis 5 mm, 0,1 mm, bis 3 mm, 0,1 mm bis 2 mm, 0,1 mm bis 1 mm, 0,1 mm bis 0,5 mm liegt.

7. Bewertungssystem (1) für intrakoronaren Fluss, umfassend:

Den Koronardraht nach irgendeinem der Ansprüche 1 bis 6, um lokale Daten der intrakoronaren Kammer und Gefäße aus einer intrakoronaren Stelle im Körper eines Patienten bereitzustellen;
einen erlernten Datensatz, umfassend: Daten von intrakoronaren Kammer und Gefäßen gegen Daten intrakoronaren Flusses;
einen ersten Eingang (5, 6) zum Empfangen von Daten vom Koronardraht; und
einen Prozessor (2), wobei der Prozessor ausgelegt ist, die Eingabedaten der intrakoronaren Kammer und Gefäße gegen den erlernten Datensatz zu analysieren, um Flussinformation für die intrakoronare Stelle im Körper des Patienten abzuleiten, wobei die abgeleitete Flussinformation ein Werkzeug umfasst, um den intrakoronaren Fluss an der intrakoronaren Stelle im Körper des Patienten zu bewerten.

8. System nach Anspruch 7, wobei die Daten der intrakoronaren Kammer und Gefäße eins oder mehrere von folgenden sind:

EKG-Daten der elektrischen Aktivität einer intrakoronaren Kammer und/oder eines Gefäßes; und
Bilddaten einer/eines intrakoronaren Kammer und/oder Gefäßes.

9. System nach Anspruch 7 oder 8, wobei die Daten des intrakoronaren Flusses eins oder mehrere von folgenden sind:

Druckdaten des intrakoronaren Flusses in einer/einem intrakoronaren Kammer oder Gefäß; und
intrakoronare Dopplerdaten des Flusses in einer/einem intrakoronaren Kammer oder Gefäß.

10. System nach irgendeinem der Ansprüche 7 bis 9, wobei das System einen zweiten Eingang zum Empfangen lokaler Daten intrakoronaren Flusses von der intrakoronaren Stelle im Körper eines Patienten aufweist, und der Prozessor ausgelegt ist, die eingegebenen Flussdaten mit den eingegebenen Daten der intrakoronaren Kammer und Gefäße gegen den erlernten Datensatz zu analysieren und Flussinformation für die intrakoronare Stelle im Körper des Patienten abzuleiten.

11. System nach irgendeinem der Ansprüche 7 bis 10, wobei das System ausgelegt ist, eine Koronararterienstenose zu bewerten, wobei der Prozessor ausgelegt ist, einen Flussquotienten abzuleiten, der lokale intrakoronare Flussinformation für die intrakoronare Stelle im Körper des Patienten enthält und um ein Druckverhältnis oder -gefälle für die intrakoronare Stelle im Körper des Patienten aus den intrakoronaren Flussdaten anhand der zweiten Eingabe zu berechnen,
wobei die Schwere der Stenose mit einem Verhältnis zwischen dem berechneten Druckverhältnis oder -gefälle und dem abgeleiteten Flussquotienten korreliert wird.

12. System nach Anspruch 11, wobei der Prozessor ausgelegt ist, die Schwere der Stenose zu berechnen als: entweder das Druckverhältnis geteilt durch den Flussquotienten oder das Druckgefälle geteilt durch den Flussquotienten.

13. System nach irgendeinem der Ansprüche 7 bis 12, wobei der Prozessor ausgelegt ist, einen Flussquotienten, der lokale intrakoronare Flussinformation für die intrakoronare Stelle im Körper des Patienten enthält, anhand der lokalen Daten für die intrakoronare Kammer und Gefäße abzuleiten.

14. System nach irgendeinem der Ansprüche 11 bis 13, wobei der Prozessor ausgelegt ist, den Flussquotienten oder Bereich von Flussquotienten abzuleiten durch: Kategorisieren der erlernten Daten in eine oder mehrere Kategorien, wobei jede Kategorie einen zugehörigen Flussquotienten oder Bereich von Flussquotienten aufweist; und

Anpassen der Daten für die intrakoronare Kammer und Gefäße an erlernte Daten in einer Kategorie; und
Zuordnen des zu jener Kategorie gehörenden Flussquotienten zu den lokalen Daten für die intrakoronare Kammer und Gefäße aus der intrakoronaren Stelle im Körper des Patienten.

15. System nach irgendeinem der Ansprüche 7 bis 14, wobei die Daten in Form von einer Wellenform sind und der erlernte Datensatz an der Wellenform ganz oder teilweise davon trainiert wird und den Eingängen zum Analysator abgetastete Daten in Form einer Wellenform derart eingespeist werden, dass Analyse auf Basis der Wellenformen stattfindet.

**Revendications**

1. Un fil métallique coronaire (10) destiné à être inséré à travers un cathéter (11) dans un vaisseau coronaire d'un patient, le fil métallique ayant :

   une longueur de travail (22) s'étendant à partir d'une extrémité proximale (18) destinée à une connexion avec un connecteur à une extrémité distale (14) destinée à une insertion à travers un cathéter dans un vaisseau coronaire d'un patient ;
   une longueur souple (23) dont une partie est exposée (12), lors de l'utilisation, à partir du cathéter et apte, lors de l'utilisation, à entrer en contact avec le vaisseau coronaire ;
   une pointe distale (17) à l'extrémité distale ;
   une électrode (7) sur la longueur souple au niveau de la pointe distale ou décalée par rapport à la pointe distale par un espacement prédéterminé ;
   un capteur de pression (13) situé dans l'électrode ; et
   une isolation électrique (21) isolant électriquement la partie exposée de la longueur souple du fil métallique excepté au niveau de l'électrode afin de permettre à l'électrode d'entrer en contact électrique avec le vaisseau coronaire.

2. Le fil métallique selon la revendication 1, en combinaison avec le cathéter.

3. Le fil métallique selon l'une quelconque des revendications précédentes, dans lequel :

   l'électrode est décalée par rapport à la pointe distale par un espacement prédéterminé dans la plage de 0 mm à 100 mm, 5 mm à 56 mm, 5 mm à 36 mm, 5 mm à 26 mm, 5 mm à 16 mm, 10 mm à 16 mm ; et/ou
   le fil métallique a plusieurs électrodes ; et/ou
   l'électrode est unique ou une combinaison de : une électrode à pointe bombée, une électrode à pointe plate, une électrode à plaque, une électrode annulaire entourant le fil métallique au moins partiellement et/ou une électrode annulaire entourant le fil.

4. Le fil métallique selon l'une quelconque des revendications précédentes, dans lequel l'isolation électrique est un revêtement sur le fil métallique ou un manchon sur le fil métallique.

5. Le fil métallique selon l'une quelconque des revendications précédentes, dans lequel il y a une fenêtre dans l'isolation électrique pour accueillir une partie du capteur de pression et la partie du capteur de pression dans la fenêtre est isolée électriquement ; ou
   dans lequel il y a une fenêtre dans l'isolation électrique pour accueillir une partie du capteur de pression et la partie du capteur de pression dans la fenêtre est isolée électriquement, dans lequel il y a une isolation électrique dans la fenêtre comprenant une membrane couvrant le capteur de pression, laquelle est déformable pour transmettre la pression subie par la membrane au capteur de pression.

6. Le fil métallique selon l'une quelconque des revendications précédentes, dans lequel le fil métallique a une section transversale généralement circulaire et le diamètre du fil métallique isolé est dans la plage de 0,35 mm à 1,27 mm, 0,3 mm à 1,5 mm, 0,2 à 2 mm ; et/ou
   le fil métallique a un axe central et la longueur de l'électrode le long de l'axe central est dans la plage de 0,01 mm à 30 mm, 0,1 mm à 20 mm, 0,1 mm à 10 mm, 0,1 mm à 5 mm, 0,1 mm à 3 mm, 0,1 mm à 2 mm, 0,1 mm à 1 mm, 0,1 mm à 0,5 mm.

7. Un système d'évaluation de flux intracoronaire (1) comprenant :

   le fil métallique coronaire selon l'une quelconque des revendications 1 à 6, pour fournir des données sur le vaisseau et la chambre intracoronaires au niveau local provenant d'un emplacement intracoronaire dans le corps d'un patient ;
   un ensemble de données apprises comprenant : des données sur le vaisseau et la chambre intracoronaires formées pour des données de flux intracoronaire ;
   une première entrée (5, 6) pour recevoir les données du fil métallique coronaire ; et
   un processeur (2) configuré pour analyser les données sur le vaisseau et la chambre intracoronaires au niveau de l'entrée par rapport à l'ensemble de données apprises pour dériver des informations sur le flux à l'emplacement intracoronaire dans le corps du patient, les informations sur le flux dérivées comprenant un outil pour évaluer le flux intracoronaire à l'emplacement intracoronaire dans le corps du patient.

8. Le système selon la revendication 7, dans lequel les données sur le vaisseau et la chambre intracoronaires sont, soit les unes, soit toutes, parmi :

des données ECG de l'activité électrique d'une chambre et/ou vaisseau intracoronaires ; et
des données d'imagerie d'une chambre et/ou vaisseau intracoronaires.

9. Le système selon la revendication 7 ou 8, dans lequel les données de flux intracoronaire sont soit les unes, soit toutes, parmi :

des données de pression intracoronaire du flux dans une chambre ou un vaisseau intracoronaire ; et
des données Doppler du flux intracoronaire dans une chambre ou un vaisseau intracoronaire.

10. Le système selon l'une quelconque des revendications 7 à 9, dans lequel le système a une deuxième entrée pour recevoir des données sur le flux intracoronaire au niveau local provenant de l'emplacement intracoronaire dans le corps d'un patient et le processeur est configuré pour analyser les données de flux avec les données d'entrée sur le vaisseau et la chambre intracoronaires au niveau de l'entrée par rapport à l'ensemble de données apprises et pour dériver des informations sur le flux à l'emplacement intracoronaire dans le corps du patient.

11. Le système selon l'une quelconque des revendications 7 à 10, dans lequel le système est configuré pour évaluer une sténose d'artère coronaire, le processeur étant configuré pour dériver un quotient de flux contenant des informations sur le flux intracoronaire au niveau local à l'emplacement intracoronaire dans le corps du patient et pour calculer un rapport ou gradient de pression à l'emplacement intracoronaire dans le corps du patient à partir des données sur le flux intracoronaire provenant de la deuxième entrée,
dans lequel la gravité de la sténose est corrélée avec un rapport entre le rapport ou gradient de pression calculé et le quotient de flux dérivé.

12. Le système selon la revendication 11, dans lequel le processeur est configuré pour calculer la gravité de la sténose comme étant : soit le rapport de pression divisé par le quotient de flux, soit le gradient de pression divisé par le quotient de flux.

13. Le système selon l'une quelconque des revendications 7 à 12, dans lequel le processeur est configuré pour dériver un quotient de flux contenant des informations sur le flux intracoronaire au niveau local à l'emplacement intracoronaire dans le corps du patient à partir des données sur le vaisseau et la chambre intracoronaires au niveau local à l'emplacement intracoronaire.

14. Le système selon l'une quelconque des revendications 11 à 13, dans lequel le processeur est configuré pour dériver le quotient de flux ou une plage de quotients de flux en :

catégorisant les données apprises en une ou plusieurs catégories, chaque catégorie ayant un quotient de flux ou une plage de quotients de flux associés ; et
intégrant les données sur le vaisseau et la chambre intracoronaires à des données apprises dans une catégorie ; et
attribuant le quotient de flux associé à ladite catégorie aux données sur le vaisseau et la chambre intracoronaires au niveau local provenant de l'emplacement intracoronaire dans le corps du patient.

15. Le système selon l'une quelconque des revendications 7 à 14, dans lequel les données sont sous la forme d'une forme d'onde et l'ensemble des données apprises sont formées sur la forme d'onde dans sa totalité ou des parties de celle-ci et les entrées dans l'analyseur sont alimentées en données détectées sous la forme d'une forme d'onde de sorte que l'analyse a lieu sur la base des formes d'onde.

Figure 1

PRIOR ART

Combo Wire I

_Figure 2_

flow sensor   pressure sensor

uninsulated radiopaque tip

SlyDx™ coating   PTFE coating

electrode terminal(s) for connector and then to console

_Figure 3_

flow sensor & pressure sensor

Combo Wire II

flexible length 30cm

working length 185cm

_Figure 4A_

13

17

"A"

7

outer surface of wire

21

10

pressure sensor offset

distal tip datum

electrode offset

wire core & structure wiring to pressure sensor and electrode

_Figure 4B_

"B"

21

13

7

17

21

Figure 4C'

perspective view "C"

schematic view "C"

Figure 4C

Figure 4D

Figure 4E

domed distal tip

distal tip datum

pressure sensor offset

pressure sensor offset

electrode offset

distal tip datum

2nd electrode offset

pressure sensor offset

distal tip datum

10

Proximal

21

10mm

17

7

Wire
Including structural elements
Electrical connector cables
Core

<u>Figure 5</u>

10

Proximal

21

21

10mm

17

13

21

7

Wire
Including structural elements
Electrical connector cables
Core

Figure 6

21

7

Proximal

17

13

Wire
Including structural elements
Electrical connector cables
Core

Figure 7

Figure 8

**False Positive**
Abnormal Pressure
Normal Flow

**True Negative**
Normal Pressure
Normal Flow

normal

FLOW

**False Positive**
Abnormal Pressure
Abnormal Flow

**False Negative**
Normal Pressure
Abnormal Flow

abnormal

Pressure
Ratio or
1/gradient

abnormal

normal

Figure 9

## Figure 10

Intracoronary ECG obtained from a totally **NORMAL** RCA. No epicardial stenosis. Normal flow.
**UNOBSTRUCTED - NORMAL FLOW**

Before adenosine (baseline)

After 40 microgram
intracoronary adenosine

| | |
|---|---|
| FFR | 0.97 |
| CFR | 2.6 |
| HSR | 0.01 |
| HMR | 1.2 |

Play
Print
Options
Mode

In intracoronary pressure/Doppler analysis, totally normal pressure, flow and resistance values were observed.

EP 3 806 725 B1

EP 3 806 725 B1

# Figure 11

Intracoronary ECG recorded from mildly diseased LAD (50% proximal stenosis) with normal flow.
**OBSTRUCTED - NORMAL FLOW**

Baseline

IC-ADO

FFR 0.79
CFR 3.1

Mild proximal LAD stenosis
Near normal FFR value (0.79)
Normal Flow (CFR: 3.1)
Normal ICECG

# Figure 12

ICECG obtained from mildly diseased LAD
**OBSTRUCTED - ABNORMAL FLOW**

Baseline

IC-ADO

EP 3 806 725 B1

Figure 12'

| FFR | 0.88 |
| CFR | 1.8 |

Intracoronary pressure/Doppler: Despite a normal FFR value low CFR (flow) value is consistent with ST segment depression shown in ICECG

## Figure 13

Intracoronary ECG obtained before/after stenting of severe RCA stenosis
**UNOBSTRUCTED - ABNORMAL FLOW**

After stenting, although perfect epicardial opening was achieved, coronary slow flow was observed which was accompanied by chest pain. In this **slow flow** condition.

## Figure 13'

ICECG recorded from distal RCA showed prominent ST segment depression.

| | |
|---|---|
| FFR | 0.96 |
| CFR | 1.9 |
| HSR | 0.20 |
| HMR | 4.9 |

Intracoronary Pressure/Doppler analysis revealed normal FFR but a low CFR and a high HMR indicating microvascular injury which is in accordance with ST **depression** observed on ICECG after stenting.

EP 3 806 725 B1

# Figure 14

Intracoronary ECG obtained before and after

Before Stent

After Stent

| FFR | 1.09 |
| CFR | 1.5 |
| HSR | 0.34 |
| HMR | 4.0 |

Intracoronary Pressure-Doppler analysis revealed a normal FFR, low CFR and a high HMR in accordance with ST elevation observed on ICECG after stenting.

EP 3 806 725 B1

## Figure 15

Intracoronary ECG obtained before and after severe CXA stenosis
Initially **OBSTRUCTED - SLOW FLOW**
then post PCI **UNOBSTRUCTIVE - SLOW FLOW**

After stenting, despite having normal epicardial segment, **slow flow** developed
in the CXA. ICECG showed significant STE at that moment.

EP 3 806 725 B1

Figure 16

# Figure 16'

At Pressure and Doppler analysis: Despite having acceptable FFR value (0.95) after PCI, this patient had a low CFR and a very high HMR value indicating microvascular injury.

EP 3 806 725 B1

Figure 17

Unobstructed vessel

Obstructed vessel

## Figure 18

Normal and Abnormal Intracoronary ECG recordings

Normal ECG

Normal ECG, Indicative of ischemia

# Figure 19

Obstructed artery,ECG ischaemia and abnormal pressure interrogation

Abnormal Angiogram

Abnormal ECG

Abnormal pressure

**Figure 20**

Figure 21

Nomenclature of the ECG complex

**EP 3 806 725 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2018116723 A1